(19) Europäisches Patentamt
European Patent Office
Office européen des brevets

(11) Veröffentlichungsnummer: **0 008 047**
**B1**

# (12) EUROPÄISCHE PATENTSCHRIFT

(45) Veröffentlichungstag der Patentschrift: **04.12.85**

(21) Anmeldenummer: **79102680.0**

(22) Anmeldetag: **27.07.79**

(51) Int. Cl.[4]: **C 07 D 417/06,** C 07 C 125/065, A 61 K 31/425 // (C07D417/06, 235:32, 277:38)

(54) **Neue 5(6)-(2-Amino-5-thiazoloyl)-benzimidazol-2-carbamin-säureester und ihre Salze, ihre Herstellung und Verwendung und sie enthaltende pharmazeutische Präparate.**

(30) Priorität: **04.08.78 CH 8359/78**

(43) Veröffentlichungstag der Anmeldung: **20.02.80 Patentblatt 80/4**

(45) Bekanntmachung des Hinweises auf die Patenterteilung: **04.12.85 Patentblatt 85/49**

(84) Benannte Vertragsstaaten: **CH DE FR GB IT NL SE**

(56) Entgegenhaltungen:
**DE - A - 2 635 326**
**DE - A - 2 711 945**

**Die Akte enthält technische Angaben, die nach dem Eingang der Anmeldung eingereicht wurden und die nicht in dieser Patentschrift enthalten sind.**

(73) Patentinhaber: **CIBA-GEIGY AG, Postfach, CH-4002 Basel (CH)**

(72) Erfinder: **Srinivasachari, Rajappa, Dr., Aarey Road, Goregaon East, Bombay 400 063 (IN)**
Erfinder: **Vasudevan, Sudarsanam, Dr., Aarey Road, Goregaon East, Bombay 400 063 (IN)**

(74) Vertreter: **Zumstein, Fritz sen., Dr. et al, Dr. F. Zumstein sen. Dr. E. Assmann Dr. R. Koenigsberger Dipl.-Ing. F. Klingseisen Dr. F. Zumstein jun. Bräuhausstrasse 4, D-8000 München 2 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



BUNDESDRUCKEREI BERLIN

**Beschreibung**

Die vorliegende Erfindung betrifft neue Benzimidazole, insbesondere Benzimidazol-2-yl-carbamate, deren Herstellung, sowie pharmazeutische Präparate, die diese Verbindungen enthalten, und auch ihre Verwendung.

In der DE-A-2 635 326 werden Methyl- bzw. Äthyl [5(6)-(2-thiazoloyl)benzimidazol-2-]carbamate und in der DE-A-2 711 945 5(6)-Thiazolidin-3-yl-carbonyl-2-carbomethoxy-amino-benzimidazole mit anthelmintischer Wirksamkeit beschrieben. Demgegenüber sind die erfindungsgemäßen Benzimidazol-2-yl-carbamate durch einen 2-Amino-5-thiazoloylrest in 5-Stellung des Benzimidazolgerüstes substituiert.

Überraschenderweise wurde nun gefunden, daß die neuen Benzimidazol-2-yl-carbamate der Formel I

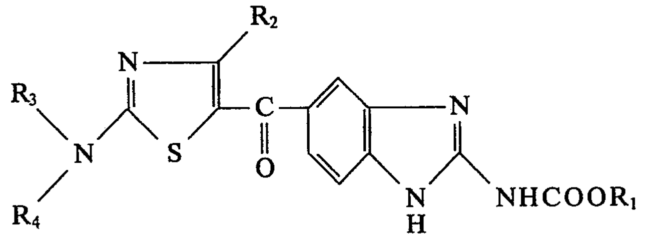

(I)

worin $R_1$ Niederalkyl, $R_2$ Wasserstoff oder Niederalkyl und $R_3$ und $R_4$ jeder für sich Wasserstoff, Niederalkyl, Niederalkenyl, Cycloalkyl oder Phenyl oder beide zusammengenommen mit dem benachbarten Stickstoffatom Niederalkylenamino bedeuten, und ihre Salze, insbesondere die pharmazeutisch verwendbaren, nicht-toxischen Salze, wobei die mit »Nieder« bezeichneten Reste bis zu 7 Kohlenstoffatome enthalten, eine ausgeprägte anthelmintische Wirksamkeit aufweisen.

$R_1$, $R_2$, $R_3$ und $R_4$ als Niederalkylgruppen sind z. B. Methyl- sowie Äthyl-, n-Propyl-, Isopropyl-, n-Butyl-, Isobutyl-, sek.-Butyl-, tert.-Butyl-, n-Pentyl-, Isopentyl-, Neopentyl-, n-Hexyl-, Isohexyl- oder n-Heptylgruppen, $R_3$ und $R_4$ als Niederalkenylgruppen sind z. B. die Allyl- oder die 2-Methylallylgruppe.

$R_3$ und $R_4$ als Cycloalkylgruppe ist z. B. eine Cyclopropyl-, Cyclobutyl-, Cyclopentyl-, Cyclohexyl- oder Cycloheptylgruppe.

Die beiden Substituenten $R_3$ und $R_4$ beide zusammengenommen können mit dem benachbarten Stickstoffatom eine Niederalkylenaminogruppe mit 4—7 Kohlenstoffatomen in der Niederalkylenkette bedeuten, in der die Niederalkylenkette beispielsweise durch ein Heteroatom substituiert sein kann.

Die Gruppe $-NR_3R_4$ in 2-Stellung des Thiazolringes ist beispielsweise Niederalkylamino oder Diniederalkylamino, z. B. Methylamino, Dimethylamino, Äthylamino, Diäthylamino, n-Propylamino, Di-n-propylamino, Isopropylamino, Di-isopropylamino oder Di-n-butylamino. Diese Gruppe bedeutet jedoch auch Niederalkylenamino, in der die Niederalkylenkette beispielsweise durch ein Heteroatom, wie z. B. Sauerstoff, Schwefel oder gegebenenfalls substituierten Stickstoff unterbrochen sein kann, und stellt z. B. Niederalkylenamino, z. B. Pyrrolidino, 2,5-Dimethylpyrrolidino, Piperidino, 2-Methylpiperidino, Hexahydroazepino oder Octahydroazocino, Oxaniederalkylenamino, z. B. Morpholino, Thianiederalkylenamino, z. B. Thiomorpholino und Azaniederalkylenamino, z. B. Piperazino, N-Methyl- oder N-Phenylpiperazino dar.

Die neuen Verbindungen besitzen wertvolle pharmakologische Eigenschaften. Sie ermöglichen einen Fortschritt in der Kontrolle von parasitären Helminthen, d. h. in der Behandlung von parasitären Infektionen durch Helminthen. Der Ausdruck »Helminthen« bezieht sich auf Nematoden, Zestoden und Trematoden, d. h. auf Würmer, die hauptsächlich den gastrointestinalen Trakt, die Leber und auch andere Organe befallen. Die Verbindungen sind hauptsächlich zur Bekämpfung von parasitären Nematoden, wie z. B. Ascaridae, Trichostrongylidae, Strongylidae und Ancylostomatidae (hookworm), und von Zestoden, wie z. B. Taeniidae und Anoplocephalidae, und Trematoden, wie z. B. Fasciolodae, besonders geeignet.

Die neuen Verbindungen beseitigen die entsprechenden Würmer in Dosen von 10 bis 100 mg/kg oral oder in Dosen von 5 bis 25 mg/kg parenteral verabreicht an Tieren, wie z. B. an Hamstern, die experimentell mit Necator-americanus oder Ancylostoma ceylanicum, oder an Hunden, die in natürlicher Weise mit Ancylostoma caninum infiziert worden sind.

Die erwähnten neuen Verbindungen sind als anthelminthisch wirksame Mittel, insbesondere als Mittel gegen Hakenwürmer (hookworm) geeignet.

Von besonderem Interesse in dieser Erfindung sind Verbindungen der Formel I, in der $R_1$ Niederalkyl, z. B. Methyl oder Äthyl, $R_2$ Niederalkyl, z. B. Methyl oder Äthyl, $R_3$ Wasserstoff und $R_4$ Niederalkyl, z. B. Methyl, Äthyl, n-Propyl, Isopropyl, n-Butyl oder Isobutyl, bedeuten, und ihre Salze, insbesondere pharmazeutisch verwendbaren, nicht-toxischen Salze.

Besonders zu nennen sind die in den Beispielen beschriebenen Verbindungen der Formel I und ihre Salze, insbesondere ihre pharmazeutisch verwendbaren, nicht-toxischen Salze.

Die neuen Benzimidazole der Formel I werden nach an sich bekannten Methoden erhalten.

So kann man z. B. die neuen Benzimidazole der Formel I erhalten, indem man ein substituiertes o-Phenylen-diaminderivat der Formel II

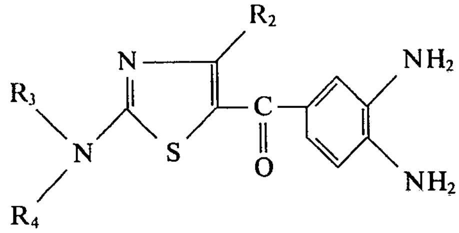

(II)

worin $R_2$, $R_3$ und $R_4$ die unter der Formel I angegebenen Bedeutungen haben, oder ein Salz davon, mit einer Verbindung der Formel III

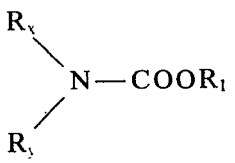

(III)

worin $R_x$ Wasserstoff oder die $COOR_1$-Gruppe und $R_y$ die Cyanogruppe bedeutet, oder $R_x$ und $R_y$ zusammengenommen eine disubstituierte Methylengruppe der Formel

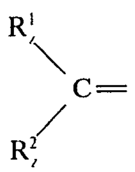

in der $R_z^1$ eine Alkylthio- oder Arylthiogruppe und $R_z^2$ eine Alkylthio- oder Arylthiogruppe, eine Amino- oder die $NHCOOR_1$-Gruppe bedeutet, umsetzt, und, wenn erwünscht, zusätzliche Verfahrensschritte durchführt.

Die im beschriebenen Verfahren zur Anwendung kommenden Verbindungen der Formel III sind beispielsweise vom Typus der Formeln

$NC—NH—COOR_1$ $NC—N(COOR_1)_2$ 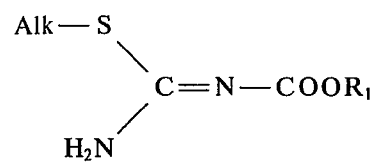

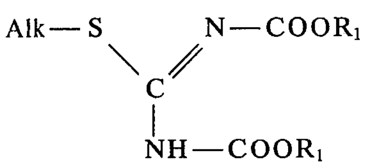 oder 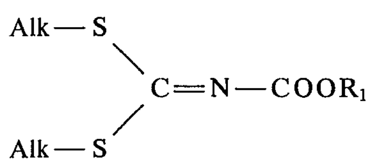

Verbindungen der Formel III werden erhalten, indem man die entsprechenden Cyanamide oder Isothioharnstoffverbindungen mit einer die Gruppe $COOR_1$ abgebenden Verbindung acyliert. Beispielsweise können monoacylierte S-Niederalkyl-isothioharnstoffe oder diacylierte S-Niederalkylisoharnstoffe, in denen der Acylrest eine $COOR_1$-Gruppe bedeutet, erhalten werden, indem man S-Niederalkyl-isothioharnstoffe mit einem oder zwei Äquivalenten eines geeigneten Halogenameisensäureesters Hal$-COOR_1$ in gekühltem, wäßrigem oder alkoholischem Medium, gegebenenfalls in Gegenwart einer Base umsetzt. Je nach Wahl kann auch das entsprechende Reaktionsgemisch in demjenigen Reaktionsmedium, in dem die folgende Umsetzung mit dem entsprechenden Thiazolyl-o-phenylendiamin-derivat bzw. Salz stattfindet, in situ erhalten werden. So erhält man z. B. durch Behandlung eines S-Niederalkyl-isothioharnstoffes mit einer Verbindung der Formel $Cl-COOR_1$ entweder mono- oder disubstituierte Derivate der Formeln

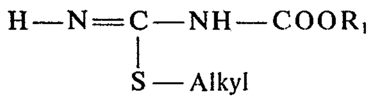 oder 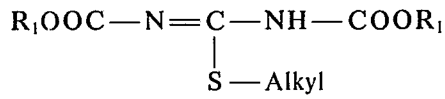

in denen $R_1$ die oben angegebene Bedeutung hat.

In einer für das beschriebene Verfahren zur Herstellung von Verbindungen der Formel I typischen Umsetzung, werden Hydrohalogenidsalze von o-Phenylendiamin-Verbindungen der Formel II mit einem oder mit zwei Äquivalenten einer Monoacyl- oder S-Diacyl-niederalkyl-isothioharnstoffverbindung, in denen Acyl eine $COOR_1$-Gruppe bedeutet, in wäßriger Lösung in Gegenwart eines Säurebindemittels, wie z. B. Natriumacetat, bei einer Temperatur von 30°C bis 200°C und einem pH-Wert von 4—6, umgesetzt. Die Umsetzung eines [Bis-(alkylthio- oder arylthio)-methylen]-amino-ameisensäurealkylesters mit einer Verbindung der Formel II erfolgt unter Erhitzen in einem inerten Lösungsmittel, wie z. B. Tetrahydrofuran oder Dioxan.

In einer weiteren Verfahrensvariante kann man o-Phenylendiamin-derivate der Formel II mit einem Mono- oder Bis-($N-R_1OOC$)-cyanamid in einer wäßrigen oder alkoholischen Lösung bei einer Temperatur von 30°C bis 200°C und einem pH-Wert von 4—9 umsetzen.

Nach einem zweiten Verfahren erhält man Verbindungen der Formel I, indem man eine Verbindung der Formel IV

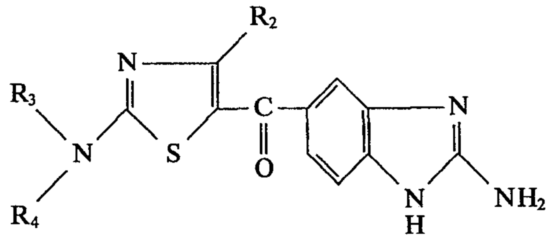

(IV)

worin $R_2$ die oben angegebenen und $R_3$ und $R_4$ mit Ausnahme von Wasserstoff die obenerwähnten Bedeutungen haben, mit einer Verbindung der Formel V

Hal—CO—X (V)

in der X ein Halogenatom oder die Gruppe $-OR_1$ bedeutet, umsetzt, und gegebenenfalls bei Verwendung einer Verbindung der Formel V, in der X Halogen bedeutet, in der erhaltenen Halogencarbonylaminoverbindung der Formel VI

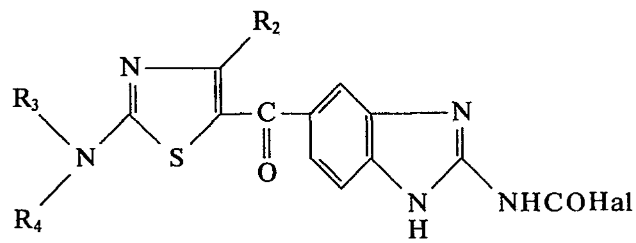

(VI)

das Halogenatom gegen die Gruppe $-OR_1$ austauscht, und, wenn erwünscht, zusätzliche Verfahrensschritte durchführt.

Für die beschriebene Umsetzung verwendet man vorzugsweise als Verbindungen der Formel V Halogenameisensäure-alkylester, insbesondere Chlorameisensäure-niederalkylester. Die Umsetzung des Aminobenzimidazolderivates der Formel IV mit einem Chlorameisensäure-niederalkylester erfolgt unter den bei dieser Reaktion üblichen Bedingungen, d. h. in Gegenwart eines geeigneten Säurebindemittels, wie z. B. einer organischen oder anorganischen Base, und eines geeigneten Lösungsmittels. Die Umsetzung wird vorzugsweise unter Erhitzen bei einer Temperatur von 50°C und Rückflußtemperatur ausgeführt.

Die beschriebene Umsetzung kann jedoch auch in zwei Schritten unter Verwendung eines Dihalogenids der Kohlensäure, vorzugsweise unter Verwendung des Dichlorids (Phosgen) und nachträglicher Umsetzung mit einem Alkohol zum Carbamat durchgeführt werden.

Nach einem dritten Verfahren erhält man Verbindungen der allgemeinen Formel I, indem man Verbindungen der allgemeinen Formel VII

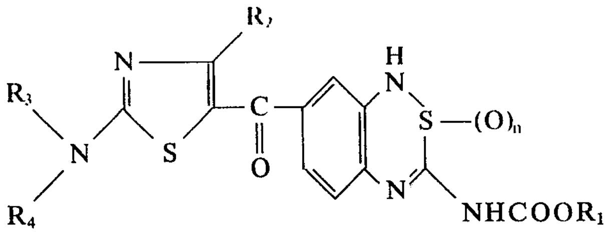

(VII)

in der $R_1$, $R_2$, $R_3$ und $R_4$ die oben angegebenen Bedeutungen haben, und n das Symbol Null oder die ganze Zahl 1 bedeutet, mit einer Säure behandelt, und gewünschtenfalls zusätzliche Verfahrensschritte durchführt.

Vorzugsweise verwendet man als Säure eine Mineralsäure, wie z. B. Schwefelsäure oder eine Halogenwasserstoffsäure, beispielsweise Salzsäure.

Die Umwandlung einer Verbindung der Formel VII durch Behandlung mit einer Mineralsäure erfolgt vorzugsweise bei erhöhter Temperatur, d. h. bei etwa 50° C bis Rückflußtemperatur, wobei, je nach der Bedeutung des Symbols n, durch Abspaltung eines Schwefelatoms oder von Schwefelmonooxid der Benzimidazolring entsteht.

Verbindungen der allgemeinen Formel I erhält man auch nach einem weiteren Verfahren, indem man einen o-Aminothioallophansäureester der Formel VIII

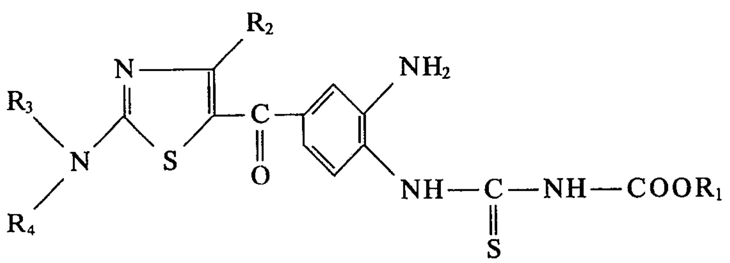

(VIII)

worin $R_1$, $R_2$, $R_3$ und $R_4$ die unter der Formel I definierten Bedeutungen haben, in Gegenwart einer Säure, vorzugsweise einer Mineralsäure, cyclisiert, und gewünschtenfalls zusätzliche Verfahrensschritte durchführt.

Die Cyclisierung eines o-Aminothioallophansäureesters der allgemeinen Formel VIII zu einem Benzimidazolderivat der Formel I wird gegebenenfalls bei erhöhter Temperatur, beispielsweise durch Erhitzen unter Rückfluß in einer wäßrigen Mineralsäure ausgeführt. Als wäßrige Mineralsäure verwendet man beispielsweise verdünnte Salzsäure.

Die erfindungsgemäßen Verbindungen der Formel I, in der einer der Reste $R_3$ und $R_4$ Wasserstoff bedeutet, und der andere die Bedeutung von $R_3$ bzw. $R_4$ hat, können nach einem weiteren Verfahren hergestellt werden, indem man in Verbindungen der Formel

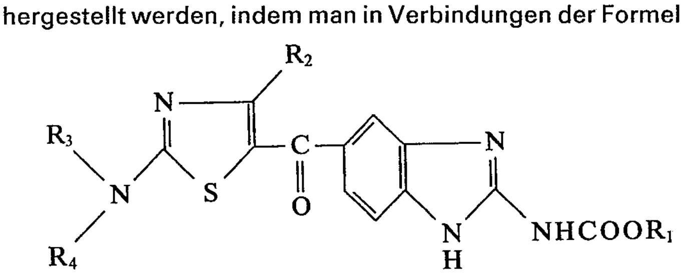

(IX)

in der $R_1$ und $R_2$ die obenerwähnten Bedeutungen haben und einer der beiden Substituenten $R_3'$ und $R_4'$ Wasserstoff ist oder die Bedeutung von $R_3$ bzw. $R_4$ hat, und der andere eine Aminoschutzgruppe bedeutet, diese abspaltet und gewünschtenfalls zusätzliche Verfahrensschritte durchführt.

Schutzgruppen $R_3'$ bzw. $R_4'$ können z. B. mittels Solvolyse oder reduktiv abgespalten werden. Solche Gruppen sind in erster Linie Acylreste, insbesondere von Kohlensäurehalbestern.

Als Aminoschutzgruppen $R_3'$ bzw. $R_4'$ kommen beispielsweise 2-Halogen-niederalkoxycarbonylgruppen, wie z. B. die 2,2,2-Trichloräthoxycarbonyl- oder die 2-Jodäthoxycarbonylgruppe in Frage, die durch Behandlung mit einem Metall oder einem entsprechenden Metallsalz, wie z. B. durch Behandlung mit Zink in wäßriger Essigsäure oder durch Behandlung mit einem Chrom-II-salz, wie z. B. Chrom-II-Clorid oder -acetat reduktiv abgespalten werden.

Weitere Aminoschutzgruppen sind geeignete Niederalkoxycarbonylgruppen, wie tert.-Butyloxycarbonyl, die acidolytisch, z. B. durch Behandeln mit Trifluoressigsäure, abspaltbar sind.

Eine weitere abspaltbare Aminoschutzgruppe ist eine Äthoxycarbonylgruppe, die in $\beta$-Stellung eine mit drei Kohlenwasserstoffresten substituierte Silylgruppe, wie Triphenylsilyl-, Dimethyl-butyl-silyl- oder vor allem Trimethylsilylgruppe, trägt. Eine solche $\beta$-(Triniederalkylsilyl)-äthoxycarbonylgruppe bildet mit der zu schützenden Aminogruppe eine entsprechende $\beta$-Triniederalkylsilyl-äthoxycarbonylaminogruppe, welche sich unter milden Bedingungen durch Einwirkung von Fluoridionen abspalten läßt. Als Fluoridionen-abgebende Reagentien kommen beispielsweise Fluoride quaternärer organischer Basen, wie Tetraäthylammoniumfluorid in Frage.

Als weitere Aminoschutzgruppe in diesem Sinne eignet sich eine gegebenenfalls durch eine Nitrogruppe substituierte Benzyloxycarbonylgruppe, wie z. B. p-Nitrobenzyloxycarbonylgruppe, die mittels Wasserstoff in Gegenwart von Palladium-auf-Kohle als Katalysator oder bei geeigneter Substitution mit Nitro durch Behandeln mit Zink und Essigsäure abgespalten werden kann.

Es ist dabei zu beachten, daß als Aminoschutzgruppen $R_3'$ und $R_4'$ nur solche in Frage kommen, die selektiv unter Erhaltung des Carbamatrestes $-NHCOOR_1$ abspaltbar sind. Ferner sollte die vorhandene Ketogruppe nicht reduziert werden.

Die beschriebenen Verfahren können in gewohnter Weise bei Raumtemperatur, unter Kühlen oder Erwärmen, bei Normaldruck oder erhöhtem Druck, und, wenn notwendig, in Gegenwart oder Abwesenheit eines Verdünnungsmittels, Katalysators oder Kondensationsmittels ausgeführt werden. Wenn notwendig, können die Umsetzungen auch in der Atmosphäre eines inerten Gases, wie z. B. Stickstoff, erfolgen.

In erhaltenen Verbindungen kann man im Rahmen der Definition der Endprodukte Substituenten einführen, abwandeln oder abspalten.

Je nach den Verfahrensbedingungen und Ausgangsstoffen erhält man die Endstoffe in freier Form oder in der ebenfalls in der Erfindung inbegriffenen Form ihrer Salze, insbesondere Säureadditionssalze. Die Säureadditionssalze der neuen Verbindungen können in an sich bekannter Weise in die freie Verbindung übergeführt werden, z. B. mit basischen Mitteln, wie Alkalien oder Ionenaustauschern. Andererseits können die erhaltenen freien Basen mit organischen oder anorganischen Säuren Salze bilden. Zur Herstellung von Säureadditionssalzen werden insbesondere solche Säuren verwendet, die zur Bildung von therapeutisch verwendbaren Salzen geeignet sind. Als solche Säuren seien beispielsweise genannt: Halogenwasserstoffsäuren, Schwefelsäuren, Phosphorsäuren, Salpetersäure, Perchlorsäure, aliphatische, alicyclische, aromatische oder heterocyclische Carbon- oder Sulfonsäuren, wie Ameisen-, Essig-, Propion-, Bernstein-, Glykol-, Milch-, Äpfel-, Wein-, Zitronen-, Ascorbin-, Malein-, Hydroxymalein- oder Brenztraubensäure; Phenylessig-, Benzoe-, p-Aminobenzoe-, Anthranil-, p-Hydroxybenzoe-, Salicyl- oder p-Aminosalicylsäure, Embonsäure, Methansulfon-, Äthansulfon-, Hydroxyäthansulfon-, Äthylensulfonsäure; Halogenbenzolsulfon-, Toluolsulfon-, Naphthalinsulfonsäure oder Sulfanilsäure; Methionin, Trypthophan, Lysin oder Arginin.

Diese oder andere Salze der neuen Verbindungen, wie z. B. die Pikrate, können auch zur Reinigung der erhaltenen freien Basen dienen, indem man die freien Basen in Salze überführt, diese abtrennt und aus den Salzen wiederum die Basen freimacht. Infolge der engen Beziehungen zwischen den neuen Verbindungen in freier Form und in Form ihrer Salze sind im vorausgegangenen und nachfolgend unter den freien Verbindungen sinn- und zweckmäßig, gegebenenfalls auch die entsprechenden Salze zu verstehen.

Die Ausgangsstoffe sind bekannt oder, falls sie neu sind, lassen sie sich nach an sich bekannten Methoden herstellen.

Ausgangsverbindungen der Formel II lassen sich in einer Folge von bekannten Reaktionsschritten, ausgehend vom 4-Halogen-3-nitro-acetophenon, herstellen. Durch Bromierung der aktiven Methylengruppe des Acetylrestes erhält man eine Verbindung der Formel X, die durch Umsetzung mit einem Thioharnstoffderivat der Formel XI

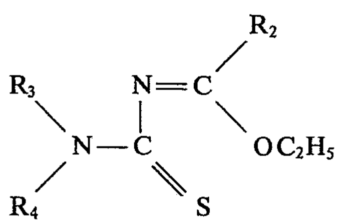

+

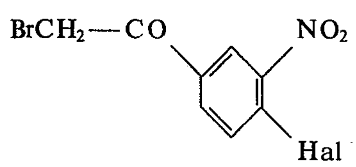

(XI) (X)

eine Verbindung der Formel XII

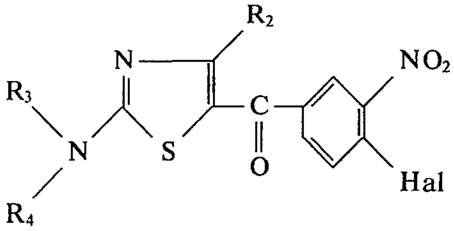

(XII)

in der $R_2$, $R_3$ und $R_4$ die unter der Formel I angegebenen Bedeutungen haben, und Hal ein Halogenatom bedeutet, ergibt.

Durch Umsetzung einer so erhaltenen Verbindung der Formel XII mit wäßrigem oder alkoholischem Ammoniak erhält man, gegebenenfalls unter Verwendung von Druck und einem Katalysator, wie z. B. Äthylenglykol, ein Nitroanilin der Formel XIII

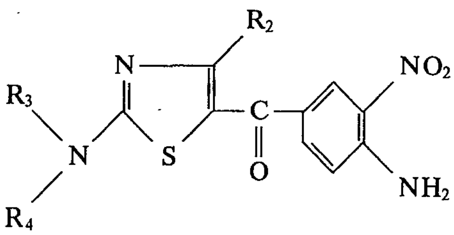

(XIII)

aus dem man wiederum durch Reduktion unter Verwendung eines geeigneten Reduktionsmittels das im ersten Verfahren als Ausgangsmaterial verwendete o-Phenylendiaminderivat der Formel II erhält.

Die Reduktion der aromatischen Nitroverbindung der Formel XIII erfolgt in an sich bekannter Weise durch Hydrierung in Gegenwart eines Palladium-auf-Kohle-Katalysators in einem geeigneten Lösungsmittel. Diese Reduktion kann auch auf chemischem Wege, wie z. B. unter Verwendung von Metallen, beispielsweise Zink, in Gegenwart von Säuren, vorzugsweise Mineralsäuren, wie z. B. Salzsäure oder auch Alkancarbonsäuren, wie z. B. Essigsäure oder Trifluoressigsäure, ausgeführt werden. Im spezifischen Falle kann auch Eisessig verwendet werden.

Die im ersten Verfahren verwendeten Bis-(alkylthio- oder -arylthio)-methylenamino-ameisensäureester der Formel

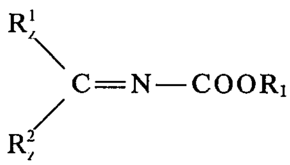

in der $R_z^1$ und $R_z^2$ eine Alkylthio- oder Arylthiogruppe bedeuten, werden hergestellt, indem man Bis-(alkylthio- oder -arylthio)-methylenamin mit einem Halogen-, insbesondere mit einem Chlorameisensäureester, umsetzt. Die auch im ersten Verfahren verwendeten und bereits vorher kurz beschriebenen monoacylierten oder bis-acylierten S-Niederalkyl-isothioharnstoffe der Formel

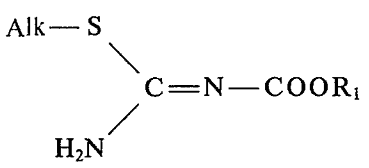

oder

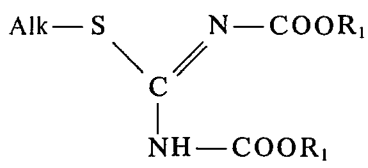

werden hergestellt, indem S-Niederalkyl-isothioharnstoffe mit einem Chlorameisensäureester in einem geeigneten Lösungsmittel, wie z. B. Wasser, acyliert und anschließend mit einer Base, wie z. B. wäßrigem Alkali, behandelt werden. Diese zur Umsetzung gelangenden Ausgangsverbindungen werden hergestellt und in situ gleichzeitig zur Umsetzung verwendet.

Die im Verfahren erwähnten Cyanamide der Formel $NC-NH-COOR_1$ können erhalten werden, indem man Cyanamid mit einem geeigneten Halogenameisensäureester, wie z. B. einem Chlorameisensäureester, umsetzt.

Die im zweiten Verfahren verwendeten Ausgangsverbindungen der Formel IV kann man erhalten, indem man ein Cyanhalogenid, insbesondere Bromcyan, mit einem o-Phenylendiamin-derivat der Formel II, wie in der Literatur beschrieben (J. Am. Chem. Soc. 69, 2459 [1947]), umsetzt.

Die im dritten Verfahren verwendeten Ausgangsverbindungen der Formel VII werden ihrerseits erhalten, indem man o-Aminothioallophansäure der Formel VIII mit einem Oxidationsmittel, wie z. B. einem Halogengas, insbesondere Brom, behandelt. Durch nachträgliche Behandlung mit einer Persäure erhält man Verbindungen der Formel VII, in der das Symbol n eine ganze Zahl 1 bedeutet.

Der im vierten Verfahren verwendete o-Amino-thioallophansäureester der Formel VIII wird hergestellt, indem man eine o-Amino-phenylendiaminverbindung der Formel II mit einem Isothiocyansäurederivat der Formel $SCN-COOR_1$ umsetzt. Die Umsetzung erfolgt vorzugsweise in einem Protonen abspaltenden Lösungsmittel, wie z. B. einer Säure, gegebenenfalls unter Anwendung von Wärme.

Die im fünften Verfahren verwendeten Ausgangsverbindungen der Formel IX werden hergestellt, indem man eine abgewandelte o-Phenylendiaminverbindung der Formel IIa

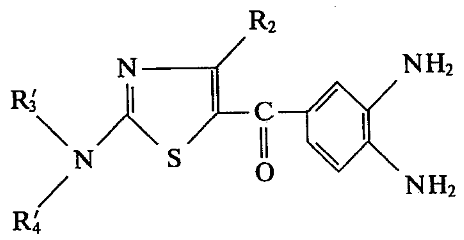

(IIa)

in der einer der Substituenten $R_3'$ und $R_4'$ Wasserstoff und der andere eine abspaltbare Aminoschutzgruppe bedeutet, mit einer Verbindung der Formel III

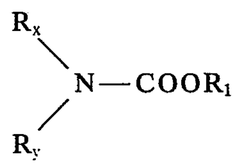

in der $R_1$, $R_x$ und $R_y$ die obenerwähnten Bedeutungen haben, umsetzt. Ausgangsverbindungen der Formel IIa werden analog wie Verbindungen der Formel II hergestellt.

Von der Erfindung ebenfalls umfaßt sind therapeutische Stoffzusammenstellungen, wie z. B. pharmazeutische Präparate, die aus einem anthelmintisch wirksamen Anteil der Verbindungen der allgemeinen Formel I oder einem Säureadditionssalz und einem pharmakologisch annehmbaren festen Trägerstoff oder flüssigen Verdünnungsmitteln bestehen, und deren Verwendung zur Herstellung von pharmazeutischen Präparaten.

Die erfindungsgemäßen pharmazeutischen Präparate enthalten mindestens eine Verbindung der allgemeinen Formel I oder ein Salz davon als Wirkstoff zusammen mit einem üblichen pharmazeutischen Trägerstoff. Die Art der Trägerstoffe richtet sich weitgehend nach dem Anwendungsgebiet. Die erfindungsgemäßen pharmazeutischen Stoffzusammensetzungen, die als Wirkstoffe Verbindungen der Formel I enthalten, können oral, parenteral oder rektal verabreicht werden.

Zur oralen Behandlung von parasitären Infektionen des Gastrointestinaltraktes, der Leber und anderer Organe kommen insbesondere feste Doseneinheitsformen, wie Tabletten, Dragées und Kapseln, in Frage, die vorzugseise zwischen 10 und 90% eines Wirkstoffes der allgemeinen Formel I oder eines Salzes enthalten, um die Verabreichung von täglichen Dosen zwischen 1,5 bis 100 mg/kg an Warmblütern zu ermöglichen. Zur Herstellung von Tabletten und Dragéekernen kombiniert man die Verbindungen der allgemeinen Formel I mit festen, pulverförmigen Trägerstoffen, wie Lactose, Saccharose, Sorbit, Maisstärke, Kartoffelstärke oder Amylopektin, Cellulosederivaten oder Gelatine, vorzugsweise unter Zusatz von Gleitmitteln, wie Magnesium- oder Calciumstearat, oder Polyäthylenglykolen von geeignetem Molekulargewicht. Dragéekerne überzieht man anschließend beispielsweise mit konzentrierten Zuckerlösungen, welche z. B. noch arabischen Gummi, Talk und/oder Titandioxid enthalten können, oder mit einem in leichtflüchtigen organischen Lösungsmitteln oder Lösungsmittelgemischen gelösten Lack. Diesen Überzügen können Farbstoffe zugefügt werden, z. B. zur Kennzeichnung verschiedener Wirkstoffdosen. Weiche Gelatinekapseln und andere geschlossene Kapseln bestehen beispielsweise aus einem Gemisch von Gelatine und Glycerin und können z. B. Mischungen einer Verbindung der Formel I mit Polyäthylenglykol enthalten. Steckkapseln enthalten z. B. Granulate eines Wirkstoffes mit festen, pulverförmigen Trägerstoffen, wie z. B. Lactose, Saccharose, Sorbit, Mannit; Stärken, wie Kartoffelstärke, Maisstärke oder Amylopektin, Cellulosederivate und Gelatine sowie Magnesiumstearat oder Stearinsäure.

Als Doseneinheitsformen für die rektale Anwendung kommen z. B. Suppositorien in Betracht, welche aus einer Kombination eines Wirkstoffes mit einer Suppositorien-Grundmasse auf der Basis von natürlichen oder synthetischen Triglyceriden (z. B. Kakaobutter), Polyäthylenglykolen oder geeigneten höheren Fettalkoholen bestehen, und Gelatine-Rektalkapseln, welche eine Kombination des Wirkstoffes mit Polyäthylenglykolen enthalten.

Ampullenlösungen zur parenteralen, insbesondere intramuskulären oder intravenösen Verabreichung enthalten eine Verbindung der Formel I oder ein Salz davon in einer Konzentration von vorzugsweise 0,5 bis 5% als wäßrige, mit Hilfe von üblichen Lösungsvermittlern und/oder Emulgiermitteln sowie gegebenenfalls von Stabilisierungsmitteln bereitete Dispersion, oder vorzugsweise eine wäßrige Lösung eines pharmazeutisch annehmbaren, wasserlöslichen Säureadditionssalzes einer Verbindung der allgemeinen Formel I.

Für Flüssigkeiten zur oralen Einnahme, wie Sirups und Elixiere, wird die Konzentration des Wirkstoffes derart gewählt, daß eine Einzeldosis leicht abgemessen werden kann, z. B. als Inhalt eines Teelöffels oder eines Meßlöffels von z. B. 5 ml oder auch als Mehrfaches dieser Volumina.

Die nachfolgenden Beispiele a) bis e) sollen die Herstellung einiger typischer Applikationsformen erläutern, jedoch keineswegs die einzigen Ausführungsformen von solchen darstellen.

a) 250,0 g Wirkstoff werden mit 550,0 g Lactose und 292,0 g Kartoffelstärke vermischt, die Mischung mit einer alkoholischen Lösung von 8 g Gelatine befeuchtet und durch ein Sieb granuliert. Nach dem Trocknen mischt man 60,0 g Talk, 10,0 g Magnesiumstearat und 20,0 g kolloidales Siliciumdioxid zu und preßt die Mischung zu 10 000 Tabletten von je 125 mg Gewicht und 25 mg Wirkstoffgehalt, die gewünschtenfalls mit Teilkerben zur feineren Anpassung der Dosierung versehen sein können.

b) Aus 100,0 g Wirkstoff, 379,0 g Lactose und der alkoholischen Lösung von 6,0 g Gelatine stellt man ein Granulat her, das man nach dem Trocknen mit 10,0 g kolloidalem Siliciumdioxid, 40,0 g Talk, 60,0 g Kartoffelstärke und 5,0 g Magnesiumstearat mischt und zu 10 000 Dragée-Kernen preßt. Diese werden anschließend mit einem konzentrierten Sirup aus 533,5 g krist. Saccharose, 20,0 g Schellack, 75,0 g arabischem Gummi, 250,0 g Talk, 20,0 g kolloidalem Siliciumdioxid und 1,5 g Farbstoff überzogen und getrocknet. Die erhaltenen Dragées wiegen je 150 mg und enthalten je 10 mg Wirkstoff.

c) 25,0 g Wirkstoff und 1975 g fein geriebene Suppositoriengrundmasse (z. B. Kakaobutter) werden gründlich gemischt und dann geschmolzen. Aus der durch Rühren homogen gehaltenen Schmelze werden 1000 Suppositorien von 2,0 g gegossen. Sie enthalten je 25 mg Wirkstoff.

d) Zur Bereitung eines Sirups mit 0,25% Wirkstoffgehalt löst man in 3 Litern dest. Wasser 1,5 Liter Glycerin, 42 g p-Hydroxybenzoesäure-methylester, 18 g p-Hydroxybenzoesäure-n-propylester und unter leichtem Erwärmen 25,0 g Wirkstoff, fügt 4 Liter 70%ige Sorbitlösung, 1000 g krist. Saccharose, 350 g Glucose und einen Aromastoff, z. B. 250 g »Orange Peel Soluble Fluid« von Eli Lilly and Co., Indianapolis, oder je 5 g natürliches Zitronenaroma und 5 g »Halb und Halb«-Essenz, beide von der Firma Haarmann und Reimer, Holzminden, Deutschland, zu, filtriert die erhaltene Lösung und ergänzt das Filtrat mit dest. Wasser auf 10 Liter.

e) Zur Bereitung einer Tropflösung mit 1,5% Wirkstoffgehalt löst man 150,0 g Wirkstoff und 30 g Natriumcyclamat in einem Gemisch von 4 Liter Äthanol (96%) und 1 Liter Propylenglykol. Andererseits mischt man 3,5 Liter 70%ige Sorbitlösung mit 1 Liter Wasser und fügt die Mischung zur obigen Wirkstofflösung. Hierauf wird ein Aromastoff, z. B. 5 g Hustenbonbon-Aroma oder 30 g Grapefruit-Essenz, beide von der Firma Haarmann und Reimer, Holzminden, Deutschland, zugegeben, das Ganze gut gemischt, filtriert und mit dest. Wasser auf 10 Liter ergänzt.

Die nachfolgenden Beispiele erläutern die Herstellung der neuen Verbindungen der allgemeinen Formel I, sollen jedoch den Umfang der Erfindung in keiner Weise beschränken. Die Temperaturen sind in Celsiusgraden angegeben.

### Beispiel 1

Das »Reagens« wird in situ wie folgt hergestellt: Ein Gemisch von 4,2 g 5-Methyl-isothioharnstoffsulfat und 5 ml Wasser wird unter Rühren auf 0—2° gekühlt und bei dieser Temperatur innerhalb von 10 Minuten tropfenweise mit 3,2 g Chlorameisensäure-äthylester behandelt. Das Reaktionsgemisch wird bei der angegebenen Temperatur 15 Minuten gerührt, dann mit 5—6 ml einer 25%igen wässerigen Natriumhydroxydlösung behandelt, bis der pH-Wert 7,5—8 erreicht, wobei man die Temperatur unter 5° hält. Das Gemisch wird bei dieser Temperatur 15 Minuten gerührt und dann tropfenweise bei 0—2° mit 2 ml Eisessig behandelt, wobei der pH-Wert auf 5—5,5 gebracht wird.

Eine Lösung von 4 g 1,2-Diamino-4-(4-methyl-2-methylamino-5-thiazoloyl)-benzol in 150 ml Methanol wird auf einmal zu dem obigen Reagensgemisch gegeben. Das Reaktionsgemisch wird unter Rühren 2 Stunden unter Rückfluß gekocht, auf 0° gekühlt, filtriert und mit 50%igem wässerigem Methanol gewaschen. Das erhaltene feste Material wird durch Auflösen in 2-normaler Chlorwasserstoffsäure und Ausfällen mit Ammoniak gereinigt und schließlich mit Wasser gewaschen. Man erhält das 2-Carbäthoxyamino-5(6)-(4-methyl-2-methylamino-5-thiazoloyl)-benzimidazol der Formel

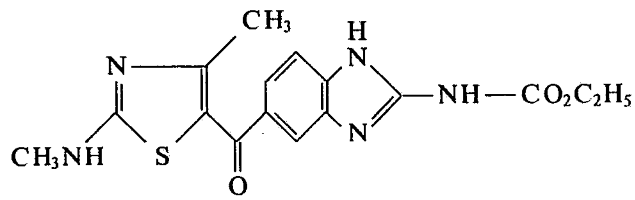

welches bei 280—285° unter Zersetzung schmilzt.

Der Ausgangsstoff für die obige Synthese wird wie folgt hergestellt: Ein Gemisch von 17 g Acetimidoäthylester und 14 g Methyl-isothiocyanat wird auf dem Wasserbad 2 Stunden erwärmt, abgekühlt und das Additionsprodukt als solches im nächsten Verfahrensschritt verwendet.

Eine Lösung von 30 g des obigen Additionsprodukts in 300 ml Isopropanol wird mit 33 g 4-Chlor-3-nitro-phenacylbromid unter Rückfluß gekocht. Nach Abkühlen wird das feste Material abfiltriert, mit Natriumhydroxyd basisch gestellt, filtriert, mit Wasser gewaschen und aus wässeriger Essigsäure umkristallisiert. Man erhält das 4-Chlor-3-nitro-1-(4-methyl-2-methylamino-5-thiazoloyl)-benzol, welches bei 230—232° schmilzt.

Ein Gemisch von 4 g der obigen Chlorverbindung und 40 ml einer alkoholischen Ammoniaklösung, die 0,2 ml Äthylenglykol enthält, wird im Bombenrohr 16 Stunden bei 130° erhitzt, abgekühlt, filtriert und der Rückstand mit Wasser gewaschen. Das feste Material wird aus wässeriger Essigsäure umkristallisiert. Man erhält das 1-Amino-2-nitro-4-(4-methyl-2-methylamino-5-thiazoloyl)-benzol, welches bei 260—261° unter Zersetzung schmilzt.

Ein Gemisch von 40 g Stannochlorid-dihydrat in 150 ml Essigsäure wird mit trockenem Chlorwasserstoffgas gesättigt und dann unter Rühren mit der obigen Nitroverbindung in kleinen Portionen versetzt. Die Lösung wird weitere 2 Stunden bei 30° gerührt und dann das Lösungsmittel im Vakuum abgedampft. Der Rückstand wird mit 50%iger wässeriger Kaliumhydroxydlösung basisch gemacht und filtriert. Der Rückstand wird mit heißem Methanol extrahiert, das Lösungsmittel abgedampft und der Rückstand mit Isopropanol digeriert. Das Produkt wird aus Methanol umkristallisiert. Man erhält das 1,2-Diamino-4-(4-methyl-2-methylamino-5-thiazoloyl)-benzol als ein hygroskopisches festes Material, das bei 115—125° schmilzt.

## Beispiel 2

Das Reagens wird in situ gemäß Beispiel 1, ausgehend von 4,5 g 5-Methyl-isothioharnstoff-sulfat und 3,5 g Chlorameisensäure-äthylester hergestellt. Die erhaltene Lösung wird mit einer Lösung von 4,5 g 1,2-Diamino-4-(4-methyl-2-äthylamino-5-thiazoloyl)-benzol in 100 ml Methanol versetzt und 2 Stunden unter Rückfluß gekocht. Das Reaktionsgemisch wird über Nacht bei 30° stehengelassen, das erhaltene feste Material abfiltriert, mit heißem Methanol gewaschen und durch Auflösen in 2-normaler Chlorwasserstoffsäure und Ausfällen mit Ammoniak gereinigt. Man erhält das 2-Carbäthoxy-5(6)-(4-methyl-2-äthylamino-5-thiazoloyl)-benzimidazol, welches bei 250—255° (Zersetzung) schmilzt.

Der Ausgangsstoff wird wie folgt hergestellt: Ein Gemisch von 20 g Acetimido-äthylester und 20 g Äthyl-isothiocyanat wird unter Rühren 2 Stunden erwärmt. Ein Gemisch von 35 g des erhaltenen rohen Additionsprodukts wird mit 56 g 4-Chlor-3-nitro-phenacylbromid in 300 ml Isopropanol 2 Stunden unter Rückfluß gekocht und gemäß Beispiel 1 aufgearbeitet. Man erhält das 4-Chlor-3-nitro-1-(4-methyl-2-äthylamino-5-thiazoloyl)-benzol, welches bei 195° schmilzt.

Ein Gemisch von 4 g der obigen Chlorverbindung, 0,4 g Äthylenglykol in 40 ml alkoholischer Ammoniaklösung wird in einem Bombenrohr 16 Stunden bei 100° erhitzt, abgekühlt, filtriert, der Rückstand mit Wasser gewaschen und aus wässeriger Essigsäure umkristallisiert. Man erhält das 1-Amino-2-nitro-4-(4-methyl-2-äthylamino-5-thiazoloyl)-benzol, welches bei 220—225° (Zersetzung) schmilzt.

Man reduziert 9 g der obigen Nitroverbindung mit 45 g Stannochlorid in 150 ml Essigsäure und arbeitet das Reaktionsgemisch gemäß Beispiel 1 auf. Man erhält das rohe 1,2-Diamino-4-(4-methyl-2-äthylamino-5-thiazolyl)-benzol, welches bei 185—225° schmilzt.

## Beispiel 3

Das Reagens wird in situ gemäß Beispiel 1, ausgehend von 8,5 g S-Methyl-isothioharnstoff-sulfat und 6,5 g Chlorameisensäure-äthylester, hergestellt. Die Lösung wird mit einer Lösung von 9 g 1,2-Diamino-4-(4-methyl-2-n-butylamino-5-thiazoloyl)-benzol in 100 ml Methanol versetzt und 4 Stunden unter Rückfluß gekocht. Nach Abkühlen wird der Niederschlag abfiltriert, mit heißem Methanol gewaschen und durch Lösen in 2-normaler Chlorwasserstoffsäure und Ausfällen mit Ammoniak gereinigt. Man erhält das 2-Carbäthoxyamino-5(6)-(4-methyl-2-n-butylamino-5-thiazoloyl)-benzimidazol-hemih-

ydrat, welches bei 208—212° (Zersetzung) schmilzt. Das wasserfreie Produkt wird durch Waschen mit heißem Methanol und Trocknen hergestellt. F. 229—233°.

Die Base wird durch Behandlung mit äthanolischem Chlorwasserstoff und Eindampfen in das Hydrochlorid umgewandelt. Eine Lösung von 4,7 g dieses Hydrochlorids in Wasser wird mit einer Lösung von 4,3 g Dinatrium-pamoat in 20 ml Wasser behandelt. Der erhaltene Niederschlag wird abfiltriert, mit Wasser gewaschen und getrocknet. Man erhält das entsprechende Pamoat, welches bei 240—245° (Zersetzung) schmilzt.

Der Ausgangsstoff wird wie folgt hergestellt: Man setzt 15 g Acetimido-äthylester mit 20 g n-Butylisothiocyanat bei 60° zwei Stunden um. Ein Gemisch von 10 g des erhaltenen Additionsprodukts und 15 g 4-Chlor-3-nitrophenacylbromid in 100 ml Isopropanol wird zwei Stunden unter Rückfluß gekocht, eingedampft, mit Wasser versetzt und gemäß Beispiel 1 aufgearbeitet. Man erhält das 4-Chlor-3-nitro-1-(4-methyl-2-n-butylamino-5-thiazoloyl)-benzol, welches bei 125—128° schmilzt.

Dieselbe Verbindung wird auch wie folgt hergestellt: 11,4 g N,N-Diäthyl-acetamidin werden mit 11,5 g n-Butyl-isothiocyanat versetzt, wobei das Gemisch exothermisch wird. Man läßt es eine Stunde bei 60° stehen, und das erhaltene Additionsprodukt wird in folgendem Versuch verwendet. Ein Gemisch von 22,9 g des rohen Produkts und 27,9 g 4-Chlor-3-nitro-phenacylbromid in 200 ml Isopropanol wird zwei Stunden unter Rückfluß gekocht. Die Lösung wird wie oben beschrieben aufgearbeitet. Man erhält das 4-Chlor-3-nitro-1-(4-methyl-2-n-butylamino-5-thiazoloyl)-benzol.

Eine Lösung von 30 g der letztgenannten Verbindung in 300 ml alkoholischem Ammoniak wird in einem Bombenrohr 20 Stunden bei 100° erhitzt, abgekühlt, filtriert und mit Wasser gewaschen. Man erhält das 1-Amino-2-nitro-4-(4-methyl-2-n-butylamino-5-thiazoloyl)-benzol, welches bei 200—205° schmilzt.

Eine Lösung von 250 g Stannochlorid-dihydrat in 500 ml konz. Chlorwasserstoffsäure wird unter Rühren innerhalb 20—30 Minuten mit 50 g der obigen Nitroverbindung portionenweise versetzt. Die Lösung wird über Nacht bei 30° gerührt und dann im Vakuum eingedampft. Der Rückstand wird in einer minimalen Menge Wasser aufgelöst, gekühlt und mit Kaliumhydroxyd basisch gemacht. Der erhaltene Niederschlag wird abfiltriert, mit Wasser gewaschen und mit heißem Essigsäureäthylester extrahiert. Der Extrakt wird konzentriert, mit Äther behandelt, abgekühlt und filtriert. Man erhält das 1,2-Diamino-4-(4-methyl-2-n-butylamino-5-thiazoloyl)-benzol, welches bei 127—130° schmilzt.

## Beispiel 4

Man reduziert 7 g 1-Amino-2-nitro-4-(4-methyl-2-isobutylamino-5-thiazoloyl)-benzol mit 40 g Stannochlorid in 150 ml Essigsäure gemäß Beispiel 1. Man erhält das ölige 1,2-Diamino-4-(4-methyl-2-isobutylamino-5-thiazoloyl)-benzol, welches in folgendem Schritt verwendet wird.

Eine Lösung von 3 g des obigen Diamins in 30 ml Methanol wird zu einer vorher (gemäß Beispiel 1) aus 2,8 g S-Methyl-isothioharnstoff-sulfat und 2,2 g Chlorameisensäure-äthylester hergestellten Reagenslösung gegeben. Die Lösung wird gerührt und unter Rückfluß drei Stunden gekocht. Das Methanol wird abdestilliert, durch 50 ml Wasser ersetzt und das Reaktionsgemisch zwei Stunden unter Rückfluß gekocht. Die Lösung wird gekühlt, filtriert, der Rückstand mit Wasser gewaschen und aus Methanol umkristallisiert. Man erhält das 2-Carbäthoxyamino-5(6)-(4-methyl-2-isobutylamino-5-thiazoloyl)-benzimidazol-hemihydrat, welches bei 178—180° (Zersetzung) schmilzt.

Der Ausgangsstoff wird wie folgt hergestellt: Ein Gemisch von 8,7 g Acetimidoäthylester und 11,5 g Isobutyl-isothiocyanat wird 4 Stunden bei 60° gehalten. Ein Gemisch von 20 g des erhaltenen rohen Additionsprodukts und 26,8 g 4-Chlor-3-nitro-phenacylbromid in 150 ml Isopropanol wird 2 Stunden unter Rückfluß gekocht und das Lösungsmittel abgedampft. Der Rückstand wird mit Natriumhydrogencarbonatlösung basisch gemacht und mit Essigsäureäthylester extrahiert. Der Extrakt wird getrocknet und eingedampft. Der Rückstand wird aus einem Gemisch von Essigsäureäthylester und Hexan umkristallisiert. Man erhält das 4-Chlor-3-nitro-1-(4-methyl-2-isobutylamino-5-thiazoloyl)-benzol, welches bei 138—142° schmilzt.

Eine Lösung von 9 g der obigen Chlorverbindung in 150 ml äthanolischem Ammoniak wird in einem Bombenrohr 12 Stunden auf 120° erhitzt, abgekühlt und das Lösungsmittel abgedampft. Der Rückstand wird mit Wasser versetzt, filtriert und getrocknet. Man erhält das 1-Amino-2-nitro-4-(4-methyl-2-isobutylamino-5-thiazoloyl)-benzol, welches bei 210—215° schmilzt.

## Beispiel 5

Die Reduktion von 5,5 g 1-Amino-2-nitro-4-(4-methyl-2-n-pentylamino-5-thiazoloyl)-benzol wird mit 25 g Stannolchlorid in 100 ml Essigsäure gemäß Beispiel 1 durchgeführt. Das erhaltene gummiartige Diamin wird in der folgenden Cyclisierung eingesetzt.

Eine Lösung von 3,8 g des obigen Diamins in 50 ml Methanol wird zu einer vorher (gemäß Beispiel 1) aus 3,4 g S-Methyl-isothioharnstoff-sulfat und 2,7 g Chlorameisensäure-äthylester hergestellten Reagenslösung gegeben. Die Lösung wird gerührt und 3 Stunden unter Rückfluß gekocht. Das Methanol

wird abdestilliert, durch 50 ml Wasser ersetzt und das Reaktionsgemisch zwei Stunden unter Rückfluß gekocht. Die Lösung wird gekühlt, filtriert, der Rückstand mit Wasser gewaschen und aus wässeriger Essigsäure umkristallisiert. Man erhält das 2-Carbäthoxyamino-5(6)-(4-methyl-2-n-pentylamino-5-thiazoloyl)-benzimidazol, welches bei 220—224° schmilzt.

Der Ausgangsstoff wird wie folgt hergestellt: Ein Gemisch von 8,7 g Acetimido-äthylester und 12,9 g n-Pentyl-isothiocyanat wird 4 Stunden bei 60° gehalten. Ein Gemisch von 21 g des erhaltenen rohen Additionsprodukts wird mit 27 g 4-Chlor-3-nitro-phenacylbromid in 150 ml Isopropanol 2 Stunden unter Rückfluß gekocht und das Lösungsmittel abgedampft. Das Produkt wird mit Natriumhydrogencarbonat basisch gemacht, mit Essigsäure-äthylester extrahiert und aus einem Gemisch von Essigsäure-äthylester und Hexan umkristallisiert. Man erhält das 4-Chlor-3-nitro-1-(4-methyl-2-n-pentylamino-5-thiazoloyl)-benzol, welches bei 99—104° schmilzt.

Eine Lösung von 9 g der obigen Chlorverbindung in 150 ml äthanolischem Ammoniak wird in einem Bombenrohr 24 Stunden auf 80° erhitzt, gekühlt und das Lösungsmittel abgedampft. Der Rückstand wird mit Wasser versetzt, filtriert, getrocknet und aus einem Essigsäure-äthylester-Hexan-Gemisch umkristallisiert. Man erhält das 1-Amino-2-nitro-4-(4-methyl-2-n-pentylamino-5-thiazoloyl)-benzol, welches bei 165—169° schmilzt.

## Beispiel 6

Das Reagens wird gemäß Beispiel 1, ausgehend von 1,4 g S-Methyl-isothioharnstoff-sulfat und 1,2 g Chlorameisensäure-äthylester, hergestellt. Die erhaltene Lösung wird mit einer Lösung von 1,8 g 1,2-Diamino-4-(4-methyl-2-allylamino-5-thiazoloyl)-benzol-dihydrochlorid in 20 ml Wasser und dann mit einer Lösung von 1,4 g Natriumacetat-trihydrat in 4 ml Wasser versetzt. Das Reaktionsgemisch wird 2 Stunden unter Rückfluß gekocht, gekühlt und filtriert. Der erhaltene Niederschlag wird mit Wasser und dann mit heißem Methanol gewaschen. Das Produkt wird durch Auflösen in 2-normaler Chlorwasserstoffsäure und Ausfällen mit Ammoniak gereinigt. Man erhält das 2-Carbäthoxyamino-5(6)-(4-methyl-2-allylamino-5-thiazoloyl)-benzimidazol, welches bei 210—219° (Zersetzung) schmilzt.

Der Ausgangsstoff wird wie folgt hergestellt: Ein Gemisch von 8,7 g Acetimido-äthylester und 10 g Allyl-isothiocyanat wird 4 Stunden bei 60° gehalten. Man kocht 2 Stunden unter Rückfluß 10 g des erhaltenen rohen Additionsprodukts mit 15 g 4-Chlor-3-nitrophenacylbromid in 80 ml Isopropanol und dampft das Lösungsmittel ab. Das Produkt wird basisch gemacht und mit Essigsäure-äthylester extrahiert. Das Lösungsmittel wird abgedampft und der Rückstand aus einem Gemisch von Essigsäure-äthylester und Hexan umkristallisiert. Man erhält das 4-Chlor-3-nitro-1-(4-methyl-2-allylamino-5-thiazoloyl)-benzol, welches bei 154—156° schmilzt.

Eine Lösung von 4,5 g der obigen Chlorverbindung in 200 ml äthanolischem Ammoniak wird 12 Stunden bei 120° in einem Bombenrohr erhitzt, abgekühlt und filtriert. Der Rückstand wird mit Wasser behandelt, getrocknet und aus wässeriger Essigsäure umkristallisiert. Man erhält das 1-Amino-2-nitro--4-(4-methyl-2-allylamino-5-thiazoloyl)-benzol, welches bei 250—252° schmilzt.

Man reduziert gemäß Beispiel 1 1,5 g der letztgenannten Nitroverbindung mit 8 g Stannochlorid-dihydrat in 50 ml Essigsäure und erhält das 1,2-Diamino-4-(4-methyl-2-allylamino-5-thiazoloyl)-benzol, welches als Dihydrochlorid isoliert wird. Das Produkt schrumpft bei 250° und schmilzt nicht bis 350°.

## Beispiel 7

Die Reduktion von 2,8 g 1-Amino-2-nitro-4-(4-methyl-2-anilino-5-thiazoloyl)-benzol wird mit 15 g Stannochlorid in 60 ml Essigsäure gemäß Beispiel 1 durchgeführt und das erhaltene ölige Diamin in der folgenden Cyclisierung verwendet.

Eine Lösung von 2,8 g des obigen Diamins in 50 ml Methanol wird zu einer vorher (gemäß Beispiel 1) aus 2,8 g S-Methyl-isothioharnstoff-sulfat und 2,2 g Chlorameisensäure-äthylester hergestellten Reagenslösung gegeben. Die Lösung wird gerührt und 3 Stunden unter Rückfluß gekocht. Das Reaktionsgemisch wird abgekühlt, filtriert, der Rückstand mit Wasser gewaschen und aus wässeriger Essigsäure umkristallisiert. Man erhält das 2-Carbäthoxyamino-5(6)-(4-methyl-2-anilino-5-thiazoloyl)-benzimidazol, welches bei 250—255° (Zersetzung) schmilzt.

Der Ausgangsstoff wird wie folgt hergestellt: Ein Gemisch von 8,7 g Acetimido-äthylester und 13,5 g Phenyl-isothiocyanat wird eine Stunde bei 60° gehalten, mit Hexan trituriert und der Rückstand abfiltriert. Man erhält das Additionsprodukt, welches bei 96—98° schmilzt. Es wird aus einem Gemisch von Essigsäure-äthylester/Hexan umkristallisiert.

Man kocht 2 Stunden unter Rückfluß 10 g des Additionsprodukts mit 13 g 4-Chlor-3-nitro-phenacylbromid in 100 ml Isopropanol. Das Lösungsmittel wird im Vakuum entfernt, der Rückstand mit Natriumhydrogencarbonatlösung basisch gemacht und mit Essigsäure-äthylester extrahiert. Der Extrakt wird getrocknet, konzentriert und mit Hexan behandelt. Man erhält das 4-Chlor-3-nitro-1-(4-methyl-2-anilino-5-thiazoloyl)-benzol, welches bei 162—165° schmilzt.

Eine Lösung von 3,5 g der obigen Chlorverbindung in 50 ml äthanolischem Ammoniak wird in einem Bombenrohr 24 Stunden auf 80° erhitzt. Die Lösung wird gekühlt, auf ein kleines Volumen konzentriert und filtriert. Der Rückstand wird mit Wasser gewaschen und aus wässeriger Essigsäure umkristallisiert. Man erhält das 1-Amino-2-nitro-4-(4-methyl-2-anilino-5-thiazoloyl)-benzol, welches bei 235—239° schmilzt.

Beispiel 8

Das Reagens wird in situ gemäß Beispiel 1 hergestellt, wobei man aber anstelle von Chlorameisensäureäthylester Chlorameisensäure-methylester verwendet: S-Methyl-isothioharnstoff-sulfat 8,4 g, Chlorameisensäure-methylester 5,7 g.

Die erhaltene Lösung wird mit einer Lösung von 9,1 g 1,2-Diamino-4-(4-methyl-2-n-butylamino-5-thiazoloyl)-benzol (welches gemäß Beispiel 3 hergestellt wird) in 200 ml Methanol versetzt und 4 Stunden unter Rückfluß gekocht. Nach Abkühlen wird der Niederschlag abfiltriert, mit heißem Methanol gewaschen und durch Auflösen in 2-normaler Chlorwasserstoffsäure und Ausfällen mit Ammoniak gereinigt. Man erhält das 2-Carbomethoxyamino-5(6)-(4-methyl-2-n-butylamino-5-thiazoloyl)-benzimidazol, welches bei 241—243° (Zersetzung) schmilzt.

Beispiel 9

Das Reagens wird gemäß Beispiel 1 in situ hergestellt, wobei man 1,7 g S-Methyl-isothioharnstoff-sulfat und 1,4 g Chlorameisensäure-äthylester verwendet. Die erhaltene Lösung wird zuerst mit einer Lösung von 2 g 1,2-Diamino-4-(4-äthyl-2-methylamino-5-thiazoloyl)-benzol-dihydrochlorid in 20 ml Wasser und dann mit einer Lösung von 1,7 g Natriumacetat-trihydrat in 5 ml Wasser versetzt. Das Reaktionsgemisch wird gerührt und 2 Stunden unter Rückfluß gekocht, abgekühlt und filtriert. Der Rückstand wird mit Wasser und dann mit heißem Methanol gewaschen und durch Lösen in 2-normaler Chlorwasserstoffsäure und Ausfällen mit Ammoniak gereinigt. Man erhält das 2-Carbäthoxyamino-5(6)-(4-äthyl-2-methylamino-5-thiazoloyl)-benzimidazol-hemihydrat, welches bei 204—208° (Zersetzung) schmilzt.

Der Ausgangsstoff wird wie folgt hergestellt: Ein Gemisch von 12 g Propionimido-äthylester und 8,4 g Methyl-isothiocyanat wird 2 Stunden bei 70° gehalten. Man kocht 2 Stunden unter Rückfluß 20,4 g des erhaltenen rohen Additionsprodukts mit 30 g 4-Chlor-3-nitro-phenacylbromid in 150 ml Isopropanol und dampft das Lösungsmittel ab. Das Produkt wird basisch gemacht und mit Methylenchlorid extrahiert. Der Extrakt wird getrocknet, eingedampft und der Rückstand aus einem Gemisch von Essigsäure-äthylester und Hexan umkristallisiert. Man erhält das 4-Chlor-3-nitro-1-(4-äthyl-2-methylamino-5-thiazoloyl)-benzol, welches bei 186—190° schmilzt.

Eine Lösung von 4 g der obigen Chlorverbindung in 100 ml äthanolischem Ammoniak wird in einem Stahlrohr 8 Stunden auf 120° erhitzt, abgekühlt und das Lösungsmittel abgedampft. Der Rückstand wird mit Wasser digeriert, filtriert und aus wässerigem Isopropanol umkristallisiert. Man erhält das 1-Amino-2-nitro-4-(4-äthyl-2-methylamino-5-thiazoloyl)-benzol, welches bei 210—215° schmilzt.

Die Reduktion von 5 g der obigen Nitroverbindung mit 25 g Stannochlorid in 100 ml Essigsäure wird gemäß Beispiel 1 durchgeführt. Das Produkt wird mit äthanolischem Chlorwasserstoff in sein Dihydrochlorid übergeführt. Nach dem Triturieren mit heißem Isopropanol erhält man das 1,2-Diamino-4-(4-äthyl-2-methylamino-5-thiazoloyl)-benzol-dihydrochlorid, welches bei 231—236° (Zersetzung) schmilzt.

Beispiel 10

Man reduziert gemäß Beispiel 1 5,5 g 1-Amino-2-nitro-4-(4-äthyl-2-äthylamino-5-thiazoloyl)-benzol mit 30 g Stannochlorid in 100 ml Essigsäure. Das Produkt wird mit Chlorwasserstoff in Isopropanol in sein Dihydrochlorid übergeführt, filtriert und gleich für die nachfolgende Cyclisierung verwendet.

Eine Lösung von 2 g des obigen Dihydrochlorids des 1,2-Diamino-4-(4-äthyl-2-äthylamino-5-thiazoloyl)-benzols in 20 ml Wasser wird zu einer vorher (gemäß Beispiel 1) aus 1,7 g S-Methyl-isothioharnstoff-sulfat und 1,4 g Chlorameisensäureäthylester hergestellten Reagenslösung gegeben und dann mit 1,7 g Natriumacetat-trihydrat in 4 ml Wasser versetzt. Das Gemisch wird gerührt und 2 Stunden unter Rückfluß gekocht, abgekühlt und filtriert. Der Rückstand wird mit Wasser und heißem Methanol gewaschen und durch Auflösen in 2-normaler Chlorwasserstoffsäure und Ausfällen mit Ammoniak gereinigt. Man erhält das 2-Carbäthoxyamino-5(6)-(4-äthyl-2-äthylamino-5-thiazoloyl)-benzimidazol, welches bei 217—220° schmilzt.

Der Ausgangsstoff wird wie folgt hergestellt: Ein Gemisch von 10 g Propionimido-äthylester und 10 g Äthyl-isothiocyanat wird 2 Stunden bei 70° erhitzt und das rohe Additionsprodukt in der nachfolgenden Reaktion verwendet. Ein Gemisch von 13,5 g des genannten Produkts und 21 g 4-Chlor-3-nitro-

phenacylbromid in 150 ml Isopropanol wird 2 Stunden unter Rückfluß gekocht, abgekühlt und filtriert. Das Filtrat wird im Vakuum eingedampft, mit Natriumhydrogencarbonatlösung basisch gemacht und mit Essigsäure-äthylester extrahiert. Der Extrakt wird getrocknet, eingedampft und der Rückstand aus einem Gemisch von Essigsäure-äthylester und Hexan umkristallisiert. Man erhält das 4-Chlor-3-nitro-1-(4-äthyl-2-äthylamino-5-thiazoloyl)-benzol, welches bei 149—152° schmilzt.

Eine Lösung von 8 g der obigen Chlorverbindung in 150 ml äthanolischem Ammoniak wird in einem Stahlrohr 14 Stunden bei 120° erhitzt. Das Reaktionsgemisch wird abgekühlt und das Lösungsmittel abgedampft. Der Rückstand wird mit Wasser digeriert, filtriert, getrocknet und aus einem Gemisch von Essigsäure-äthylester und Hexan umkristallisiert. Man erhält das 1-Amino-2-nitro-4-(4-äthyl-2-äthylamino-5-thiazoloyl)-benzol, welches bei 210—212° schmilzt.

## Beispiel 11

Das Reagens wird in situ gemäß Beispiel 1, ausgehend von 2,8 g S-Methylisothioharnstoff-sulfat und 2,2 g Chlorameisensäure-äthylester, hergestellt. Die erhaltene Lösung wird mit einer Lösung von 2,8 g 1,2-Diamino-4-(4-methyl-2-dimethylamino-5-thiazoloyl)-benzol in 80 ml Methanol versetzt und 2 Stunden unter Rückfluß gekocht. Die Lösung wird abgekühlt, eingedampft, der Rückstand mit Wasser digeriert und filtriert. Der Rückstand wird mit heißem Methanol gewaschen und durch Auflösen in 2-normaler Chlorwasserstoffsäure und Ausfällen mit Ammoniak gereinigt. Man erhält das 2-Carbäthoxyamino-5(6)-(4-methyl-2-dimethylamino-5-thiazoloyl)-benzimidazol-monohydrat, welches bei 270—275° (Zersetzung) schmilzt.

Der Ausgangsstoff wird wie folgt hergestellt: Ein Gemisch von 3,7 g N-Acetyl-N',N'-dimethyl-thioharnstoff und 7 g 4-Chlor-3-nitro-phenacylbromid in 100 ml Isopropanol wird 2 Stunden unter Rückfluß gekocht, abgekühlt und filtriert. Der Rückstand wird basisch gemacht und mit Methylenchlorid extrahiert. Der Extrakt wird getrocknet, eingedampft und der Rückstand aus einem Gemisch von Essigsäure-äthylester und Hexan umkristallisiert. Man erhält das 4-Chlor-3-nitro-1-(4-methyl-2-dimethylamino-5-thiazoloyl)-benzol, welches bei 165—169° schmilzt.

Ein Gemisch von 3,5 g der obigen Chlorverbindung und 0,5 ml Äthylenglykol in 40 ml alkoholischem Ammoniak wird in einem Bombenrohr 16 Stunden auf 130° erhitzt. Das Produkt wird filtriert, mit Wasser gewaschen und aus einem Gemisch von Essigsäure-äthylester und Hexan umkristallisiert. Man erhält das 1-Amino-2-nitro-4-(4-methyl-2-dimethylamino-5-thiazoloyl)-benzol, welches bei 225—228° schmilzt.

Es werden 7 g der obigen Nitroverbindung mit 40 g Stannochlorid in 150 ml Essigsäure gemäß Beispiel 1 reduziert. Das Produkt wird mit Methylenchlorid extrahiert, der Extrakt getrocknet und eingedampft. Der Rückstand wird aus Isopropanol umkristallisiert. Man erhält das 1,2-Diamino-4-(4-methyl-2-dimethylamino-5-thiazoloyl)-benzol, welches bei 174—178° schmilzt.

## Beispiel 12

Das Reagens wird in situ gemäß Beispiel 1, ausgehend von 2,8 g S-Methyl-isothioharnstoff-sulfat und 2,2 g Chlorameisensäure-äthylester, hergestellt. Die erhaltene Lösung wird mit einer Lösung von 3,8 g 1,2-Diamino-4-(4-methyl-2-pyrrolidinyl-5-thiazoloyl)-benzol-dihydrochlorid in 20 ml Wasser und dann mit einer Lösung von 2,8 g Natriumacetat-trihydrat in 8 ml Wasser versetzt. Das Reaktionsgemisch wird 2 Stunden gerührt und unter Rückfluß gekocht, abgekühlt und filtriert. Der Rückstand wird zuerst mit Wasser und dann mit heißem Methanol gewaschen, durch Auflösen in 2-normaler Chlorwasserstoffsäure und Ausfällen mit Ammoniak gereinigt. Man erhält das 2-Carbäthoxyamino-5(6)-(4-methyl-2-pyrrolidinyl-5-thiazoloyl)-benzimidazol-hemihydrat, welches bei 185—195° (Zersetzung) schmilzt.

Der Ausgangsstoff wird wie folgt hergestellt: Ein Gemisch von 10 g N-Acetyl-dithiocarbaminsäuremethylester und 5 g Pyrrolidin in 100 ml Äthanol wird 16 Stunden bei 30° gehalten, das Lösungsmittel im Vakuum abgedampft und der Rückstand aus einem Gemisch von Essigsäureäthylester und Hexan umkristallisiert. Man erhält das Pyrrolidinothiocarbonyl-acetamid, welches bei 110—120° schmilzt.

Ein Gemisch des letztgenannten Thioharnstoffderivats und 16,8 g 4-Chlor-3-nitro-phenacylbromid wird in 200 ml Isopropanol 3 Stunden unter Rückfluß gekocht und eingedampft. Der Rückstand wird mit Wasser digeriert, mit Natriumhydrogencarbonatlösung basisch gemacht und mit Essigsäure-äthylester extrahiert. Der Extrakt wird getrocknet, konzentriert, gekühlt und filtriert. Man erhält das 4-Chlor-3-nitro-1-(4-methyl-2-pyrrolidinyl-5-thiazoloyl)-benzol, welches bei 168—171° schmilzt.

Eine Lösung von 4 g der obigen Chlorverbindung in 100 ml äthanolischem Ammoniak wird in einem Stahlrohr 8 Stunden bei 120° erhitzt, abgekühlt und filtriert. Der Rückstand wird mit Wasser gewaschen und aus wässeriger Essigsäure umkristallisiert. Man erhält das 1-Amino-2-nitro-4-(4-methyl-2-pyrrolidinyl-5-thiazoloyl)-benzol, welches bei 245—248° (Zersetzung) schmilzt.

Man reduziert gemäß Beispiel 1 6 g der obigen Nitroverbindung mit 30 g Stannochlorid in 100 ml Essigsäure. Das Produkt wird mit Chlorwasserstoff in Äthanol in sein Hydrochlorid übergeführt, mit heißem Isopropanol digeriert und filtriert. Man erhält das 1,2-Diamino-4-(4-methyl-2-pyrrolidinyl-5-thiazoloyl)-benzol-dihydrochlorid, welches bei 210—220° (Zersetzung) schmilzt.

Beispiel 13

Man reduziert 4 g 1-Amino-2-nitro-4-(2-n-butylamino-5-thiazoloyl)-benzol mit 20 g Stannochlorid in 100 ml Essigsäure gemäß Beispiel 1. Das erhaltene rohe Diamin wird in der nachfolgenden Cyclisierung verwendet.

Eine Lösung von 4 g des obigen Diamins in 50 ml Methanol wird zu einer vorher (gemäß Beispiel 1) aus 4,2 g S-Methyl-isothioharnstoff-sulfat und 3,2 g Chlorameisensäure-äthylester hergestellten Reagenslösung gegeben. Die Lösung wird gerührt und 3 Stunden unter Rückfluß gekocht. Das Methanol wird abdestilliert, durch 50 ml Wasser ersetzt und das Reaktionsgemisch 2 Stunden unter Rückfluß gekocht. Die Lösung wird abgekühlt, filtriert und der Niederschlag mit Wasser gewaschen. Das Produkt wird durch Auflösen in 2-normaler Chlorwasserstoffsäure und Ausfällen mit Ammoniak gereinigt. Man erhält das 2-Carbäthoxyamino-5(6)-(2-n-butylamino-5-thiazoloyl)-benzimidazol, welches bei 295—300° (Zersetzung) schmilzt.

Der Ausgangsstoff wird wie folgt hergestellt: Ein Gemisch von 5 g N-Butyl-thioharnstoff und 15 ml Dimethylformamid-dimethylacetal wird 6 Stunden unter Rückfluß gekocht und der Überschuß an DMF-acetal im Vakuum abgedampft. Der Rückstand wird in der folgenden Kondensation verwendet.

Man läßt 7 g des erhaltenen Imidoylthioharnstoffs mit 11 g 4-Chlor-3-nitro-phenacylbromid in 100 ml Isopropanol 1 Stunde unter Rückfluß kochen. Das Lösungsmittel wird abgedampft, der Rückstand mit Wasser und Essigsäureäthylester digeriert und filtriert. Das feste Material wird aus Essigsäure-äthylester umkristallisiert. Man erhält das 4-Chlor-3-nitro-1-(2-n-butylamino-5-thiazoloyl)-benzol, welches bei 192—194° schmilzt.

Man nimmt 8 g der obigen Chlorverbindung und erhitzt sie 24 Stunden in einem Stahlrohr mit 150 ml äthanolischem Ammoniak bei 80°. Das Reaktionsgemisch wird abgekühlt, filtriert, der Rückstand mit Wasser gewaschen, getrocknet und aus Essigsäure-äthylester umkristallisiert. Man erhält das 1-Amino-2-nitro-4-(2-n-butylamino-5-thiazoloyl)-benzol, welches bei 198—203° schmilzt.

Beispiel 14

Man reduziert gemäß Beispiel 1 5 g 1-Amino-2-nitro-4-(2-cyclohexylamino-5-thiazoloyl)-benzol mit 25 g Stannochlorid in 125 ml Essigsäure. Das erhaltene rohe Diamin wird in der folgenden Cyclisierung verwendet.

Eine Lösung von 4,5 g des obigen Diamins in 50 ml Methanol wird zu einer vorher (gemäß Beispiel 1) aus 4,2 g S-Methylisothioharnstoff-sulfat und 3,2 g Chlorameisensäure-äthylester hergestellten Reagenslösung gegeben. Die Lösung wird gerührt und unter Rückfluß 2 Stunden gekocht. Das Methanol wird dann abdestilliert, durch 50 ml Wasser ersetzt und das Gemisch wieder 2 Stunden unter Rückfluß gekocht. Die Lösung wird gekühlt, filtriert, der Rückstand mit Wasser gewaschen und durch Auflösen in 2-normaler Chlorwasserstoffsäure und Ausfällen mit wässerigem Ammoniak gereinigt. Der Niederschlag wird abfiltriert, mit Wasser gewaschen, getrocknet, mit heißem Essigsäureäthylester digeriert und wieder abfiltriert. Man erhält das 2-Carbäthoxyamino-5(6)-(2-cyclohexylamino-5-thiazoloyl)-benzimidazol, welches bei 216—219° (Zersetzung) schmilzt.

Der Ausgangsstoff wird wie folgt hergestellt: Ein Gemisch von 5 g N-Cyclohexylthioharnstoff und 10 ml Dimethylformamid-dimethylacetal wird 6 Stunden unter Rückfluß gekocht und der Überschuß an DMF-acetal im Vakuum entfernt. Der Rückstand wird mit Hexan digeriert und das feste Material aus einem Gemisch von Essigsäure-äthylester und Hexan umkristallisiert. Man erhält das N,N-Dimethyl-N'-cyclohexyl-thiocarbamyl-formamidin, welches bei 116—120° schmilzt.

Man läßt 5 g der letztgenannten Verbindung mit 7 g 4-Chlor-3-nitro-phenacylbromid in 100 ml Isopropanol 2 Stunden unter Rückfluß kochen. Das Lösungsmittel wird abgedampft, der Rückstand mit Wasser und Äther digeriert, filtriert und aus Essigsäure-äthylester umkristallisiert. Man erhält das 4-Chlor-3-nitro-1-(2-cyclohexylamino-5-thiazoloyl)-benzol, welches bei 194—198° schmilzt.

Eine Lösung von 6 g der letztgenannten Verbindung in 150 ml äthanolischem Ammoniak wird in einem Stahlrohr 24 Stunden auf 80° erhitzt, abgekühlt und filtriert. Der Rückstand wird mit Wasser gewaschen und aus wässeriger Essigsäure umkristallisiert. Man erhält das 1-Amino-2-nitro-4-(2-cyclohexylamino-5-thiazoloyl)-benzol, welches bei 245—250° schmilzt.

**Patentansprüche**

1. Benzimidazolcarbamate der Formel I

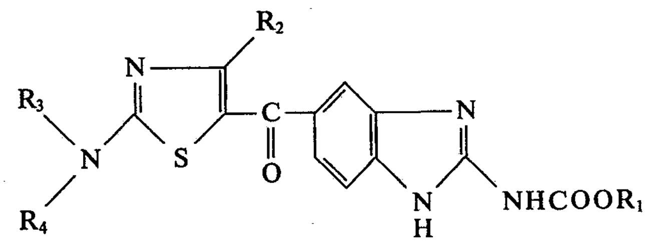

(I)

worin $R_1$ Niederalkyl, $R_2$ Wasserstoff oder Niederalkyl, und $R_3$ und $R_4$ jeder für sich Wasserstoff, Niederalkyl, Niederalkenyl, Cycloalkyl oder Phenyl, oder beide zusammengenommen mit dem benachbarten Stickstoffatom Niederalkylenamino bedeuten, und ihre Salze, insbesondere die pharmazeutisch verwendbaren, nicht-toxischen Salze, wobei die mit »Nieder« bezeichneten Reste bis zu 7 Kohlenstoffatome enthalten.

2. Verbindungen der Formel I gemäß Anspruch 1, in der $R_1$ und $R_2$ Niederalkyl, $R_3$ Wasserstoff und $R_4$ Niederalkyl bedeuten, und ihre Salze, insbesondere pharmazeutisch verwendbaren, nicht-toxischen Salze, wobei die mit »Nieder« bezeichneten Reste bis zu 7 Kohlenstoffatome enthalten.

3. 2-Carbäthoxyamino-5(6)-(4-methyl-2-n-butylamino-5-thiazoloyl)-benzimidazol und seine pharmazeutisch verwendbaren Salze.

4. 2-Carbomethoxyamino-5(6)-(4-methyl-2-n-butylamino-5-thiazoloyl)-benzimidazol und seine pharmazeutisch verwendbaren Salze.

5. Pharmazeutische Präparate mit anthelminthischen Eigenschaften, gekennzeichnet durch einen Gehalt an einer unter der allgemeinen Formel I genannten Verbindungen gemäß den Ansprüchen 1—6 in Kombination mit geeignetem Trägerstoff und/oder Verteilungsmittel.

6. Die Verbindungen der Ansprüche 1—4 zur Verwendung als Anthelminthika.

7. Verwendung der Verbindungen der Ansprüche 1—4 zur Herstellung von pharmazeutischen Präparaten.

8. Verfahren zur Herstellung von Benzimidazolcarbamaten der im Anspruch 1 angegebenen allgemeinen Formel I, worin alle Symbole die im Anspruch 1 angegebenen Bedeutungen haben, ihre tautomeren Verbindungen und Salze, dadurch gekennzeichnet, daß man

a) ein substituiertes o-Phenylendiaminderivat der Formel II

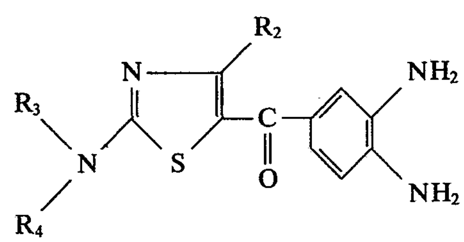

(II)

worin $R_2$, $R_3$ und $R_4$ die unter der Formel I angegebenen Bedeutungen haben, oder ein Salz davon, mit einer Verbindung der Formel III

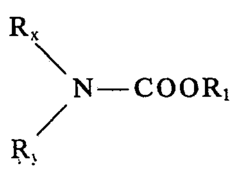

(III)

worin $R_x$ Wasserstoff oder die $COOR_1$-Gruppe und $R_y$ die Cyanogruppe bedeutet oder $R_x$ und $R_y$ zusammengenonmmen eine disubstituierte Methylengruppe der Formel

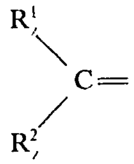

in der $R_z^1$ eine Alkylthio- oder Arylthiogruppe und $R_z^2$ eine Alkylthio- oder Arylthiogruppe, eine Amino- oder die $NHCOOR_1$-Gruppe bedeutet, umsetzt, oder

b) eine Verbindung der Formel IV

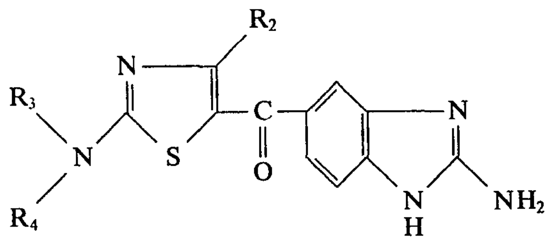

(IV)

worin $R_2$ die oben gegebenen und $R_3$ und $R_4$ mit Ausnahme von Wasserstoff die obenerwähnten Bedeutungen haben, mit einer Verbindung der Formel V

$Hal-CO-X$ (V)

in der X ein Halogenatom oder die Gruppe $-OR_1$ bedeutet, umsetzt, und gegebenenfalls bei Verwendung einer Verbindung der Formel V, in der X Halogen bedeutet, in der erhaltenen Halogencarbonylaminoverbindung der Formel VI

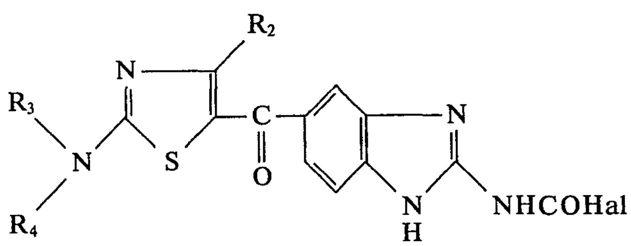

(VI)

das Halogenatom gegen die Gruppe $-OR_1$ austauscht, oder

c) Verbindungen der Formel VII

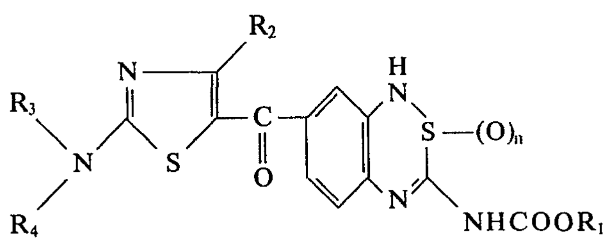

(VII)

in der $R_1$, $R_2$, $R_3$ und $R_4$ die oben angegebenen Bedeutungen haben, und n das Symbol Null oder die ganze Zahl 1 bedeutet, mit einer Säure behandelt, oder

d) einen o-Aminothioallophansäureester der Formel VIII,

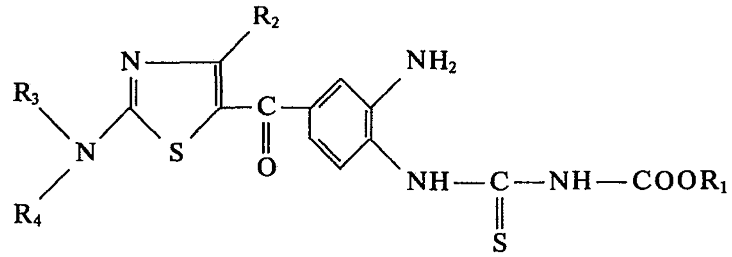

(VIII)

worin $R_1$, $R_2$, $R_3$ und $R_4$ die unter der Formel I definierten Bedeutungen haben, in Gegenwart einer Säure, vorzugsweise einer Mineralsäure, cyclisiert, oder

e) in Verbindungen der Formel IX

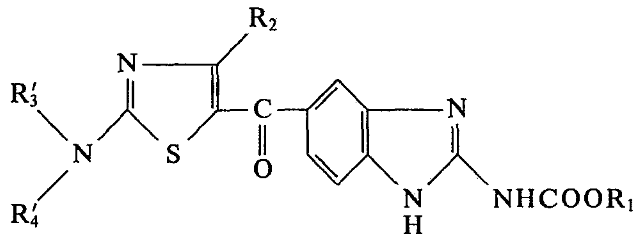

(IX)

in der $R_1$ und $R_2$ die obenerwähnten Bedeutungen haben und einer der beiden Substituenten $R_3'$ und $R_4'$ Wasserstoff ist oder die Bedeutung von $R_3$ bzw. $R_4$ hat und der andere eine Aminoschutzgruppe bedeutet, diese abspaltet, und, wenn erwünscht, eine erhaltene Verbindung der Formel I in eine andere Verbindung der Formel I überführt, und/oder, wenn erwünscht, ein erhaltenes Salz in die freie Verbindung oder in ein anderes Salz überführt, und/oder, wenn erwünscht, eine erhaltene freie Verbindung in ein Salz überführt, und/oder, wenn erwünscht, ein erhaltenes Gemisch von Isomeren in die einzelnen Isomeren auftrennt.

9. Die nach dem Verfahren des Anspruchs 8 erhältlichen Verbindungen.

**Claims**

1. A benzimidazole carbamate of formula I

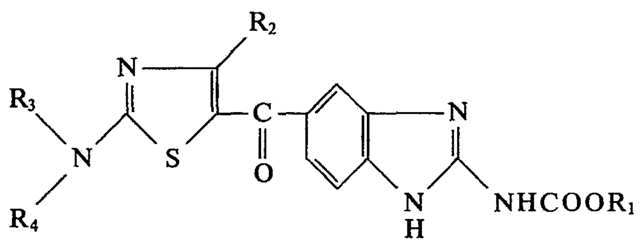

(I)

wherein $R_1$ is lower alkyl, $R_2$ is hydrogen or lower alkyl and each of $R_3$ and $R_4$ is hydrogen, lower alkyl, lower alkenyl, cycloalkyl or phenyl, or $R_3$ and $R_4$ together with the adjacent nitrogen atom are lower alkyleneamino, or a salt thereof, in particular a pharmaceutically acceptable non-toxic salt, with the radicals qualified by the term »lower« containing up to 7 carbon atoms.

2. A compound of formula I according to claim 1, wherein $R_1$ and $R_2$ are lower alkyl, $R_3$ is hydrogen und $R_4$ is lower alkyl, or a salt thereof, in particular a pharmaceutically acceptable non-toxic salt, with the radicals qualified by the term »lower« containing up to 7 carbon atoms.

3. 2-Carboethoxyamino-5(6)-(4-methyl-2-n-butylamino-5-thiazolyl)-benzimidazole or a pharmaceutically acceptable salt thereof.

4. 2-Carbomethoxyamino-5(6)-(4-methyl-2-n-butylamino-5-thiazolyl)benzimidazole or a pharmaceutically acceptable salt thereof.

5. A pharmaceutical composition having anthelmintic properties, which composition contains a compound of the general formula I according to any one of claims 1 to 4, together with a suitable carrier and/or dispersant.

6. Use of a compound according to any one of claims 1 to 4 as anthelmintic.

7. Use of a compound according to any one of claims 1 to 4 for the preparation of a pharmaceutical composition.

8. A process for the preparation of a benzimidazole carbamate of the general formula I according to claim 1, wherein all symbols are as defined in claim 1, or of a tautomer or salt thereof, which process comprises

a) reacting a substituted o-phenylenediamine derivative of formula II

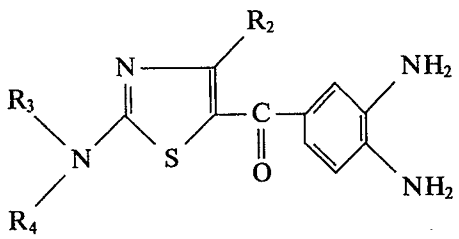

(II)

wherein $R_2$, $R_3$ and $R_4$ are as defined for formula I, or a salt thereof, with a compound of formula III

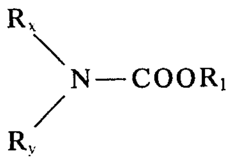

(III)

wherein $R_x$ is hydrogen or the $COOR_1$ group and $R_y$ is the cyano group or $R_x$ and $R_y$ together are a disubstituted methylene group of the formula

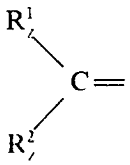

in which $R_z^1$ is an alkylthio or arylthio group and $R_z^2$ is an alkylthio or arylthio group, an amino group or the $NHCOOR_1$ group, or

b) reacting a compound of formula IV

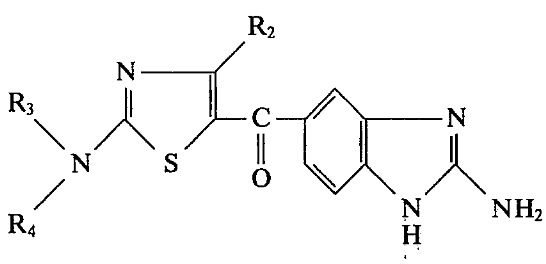

(IV)

wherein $R_2$ is as defined above and $R_3$ and $R_4$ are as defined above, with the exception of hydrogen, with a compound of formula V

Hal—CO—X (V)

wherein X is a halogen atom or the $-OR_1$ group, and, if desired, when using a compound of formula V, wherein X is halogen, replacing in the resultant halocarbonylamino compound of formula VI

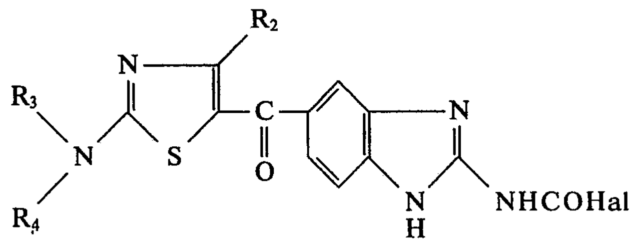

(VI)

the halogen atom by the $-OR_1$ group, or

c) treating a compound of formula VII

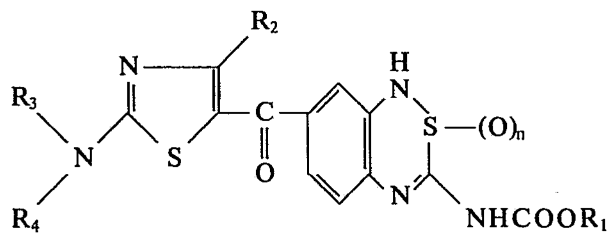

(VII)

wherein $R_1$, $R_2$, $R_3$ and $R_4$ are as defined above and n is zero or the integer 1, with an acid, or

d) cyclising an o-aminothioallophanate of formula VIII

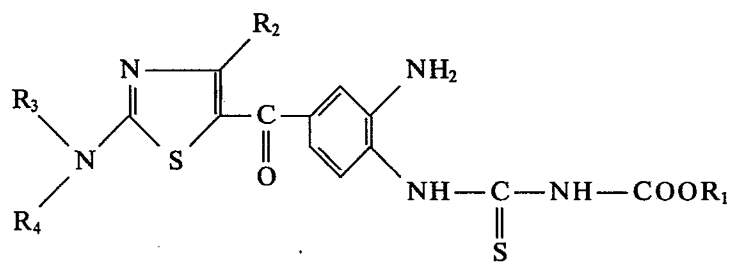

(VIII)

wherein $R_1$, $R_2$, $R_3$ and $R_4$ are as defined for formula I, in the presence of an acid, preferably a mineral acid, or

e) in a compound of formula IX

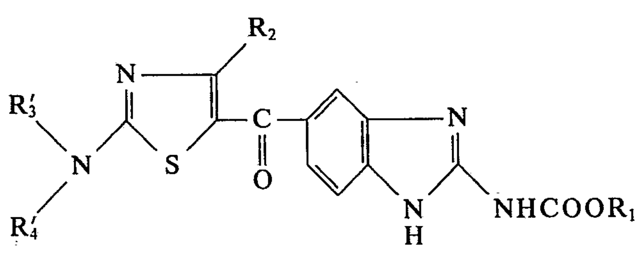

(IX)

wherein $R_1$ and $R_2$ are as defined above and one of $R_3'$ and $R_4'$ is hydrogen or has the meaning of $R_3$ or $R_4$ and the other is an amino protecting group, removing said protecting group; and, if desired, converting a resultant compound of formula I into another compound of formula I, and/or, if desired, converting a resultant salt into the free compound or into another salt, and/or, if desired, converting a resultant free compound into a salt, and/or, if desired, separating a resultant mixture of isomers into the individual isomers.

9. A compound obtainable by the process according to claim 8.

**Revendications**

1. Benzimidazole-carbamate de formule I

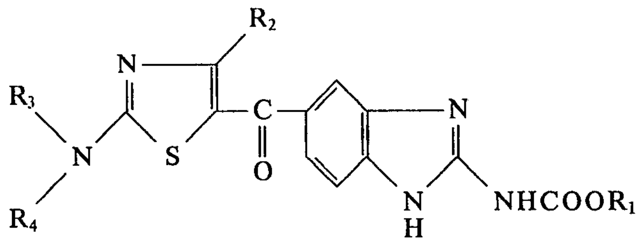

(I)

dans laquelle $R_1$ représente un groupe alkyle inférieur, $R_2$ l'hydrogène ou un groupe alkyle inférieur, et $R_3$ et $R_4$ représentent chacun un atome d'hydrogène, un groupe alkyle inférieur, alcényle inférieur, cycloalkyle ou phényle, ou bien formant ensemble et avec l'atome d'azote voisin un groupe alkylène-amino inférieur, et leurs sels, en particulier leurs sels non toxiques acceptables pour l'usage pharmaceutique, les groupes qualifiés d'»inférieurs« contenant jusqu'à 7 atomes de carbone.

2. Composés de formule I selon la revendication 1, dans lesquels $R_1$ et $R_2$ représentent des groupes alkyle inférieurs, $R_3$ l'hydrogène et $R_4$ un groupe alkyle inférieur, et leurs sels, en particulier leurs sels non toxiques acceptables pour l'usage pharmaceutique, les restes qualifiés d'»inférieurs« contenant jusqu'à 7 atomes de carbone.

3. Le 2-carbéthoxyamino-5(6)-(4-méthyl-2-n-butylamino-5-thiazoloyl)-benzimidazole et ses sels acceptables pour l'usage pharmaceutique.

4. Le 2-carbométhoxyamino-5(6)-(4-méthyl-2-n-butylamino-5-thiazoloyl)-benzimidazole et ses sels acceptables pour l'usage pharmaceutique.

5. Compositions pharmaceutiques à propriétés antihelminthiques, caractérisées en ce qu'elles contiennent un des composés de formule générale I selon les revendications 1 à 6, en combinaison avec un véhicule et/ou diluant approprié.

6. Les composés des revendications 1 à 4, pour l'utilisation en tant qu'antihelminthiques.

7. L'utilisation des composés des revendications 1 à 4 pour la préparation de compositions pharmaceutiques.

8. Procédé de préparation des benzimidazolecarbamates de formule générale I de la revendication 1, dans laquelle tous les symboles ont les significations indiquées dans la revendication 1, de leurs composés tautomères et sels, caractérisé en ce que:

a) on fait réagir une o-phénylène-diamine substituée de formule II

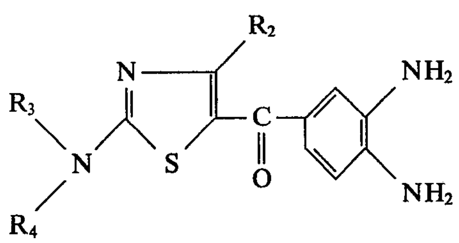

(II)

dans laquelle $R_2$, $R_3$ et $R_4$ ont les significations indiquées en référence à la formule I, ou un sel d'un tel composé, avec un composé de formule III

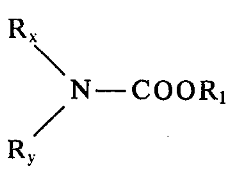

(III)

dans laquelle $R_x$ représente l'hydrogène ou le groupe $COOR_1$ et $R_y$ représente le groupe cyano, ou bien $R_x$ et $R_y$ forment ensemble un groupe méthylène disubstitué de formule

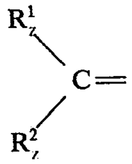

dans laquelle $R_z^1$ représente un groupe alkylthio ou arylthio et $R_z^2$ représente un groupe alkylthio ou arylthio, un groupe amino ou le groupe $NHCOOR_1$, ou bien

b) on fait réagir un composé de formule IV

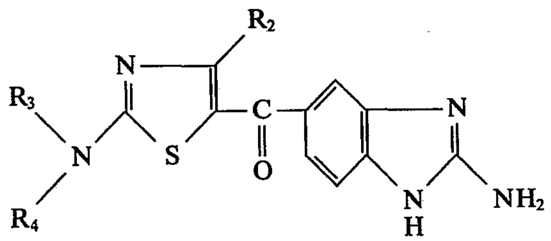

(IV)

dans laquelle $R_2$ a les significations indiquées cidessus et $R_3$ et $R_4$ ont les significations indiquées ci-dessus à l'exception de l'hydrogène, avec un composé de formule V

Hal—CO—X (V)

dans laquelle X représente un atome d'halogène ou le groupe $-OR_1$, et le cas échéant, lorsqu'on a utilisé un composé de formule V dans laquelle X représente un halogène, on échange l'atome d'halogène du composé halogénocarbonylaminé ainsi obtenu, répondant à la formule VI

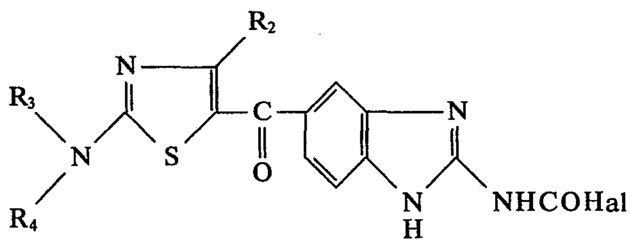

(VI)

contre le groupe $-OR_1$, ou bien

c) on traite des composés de formule VII

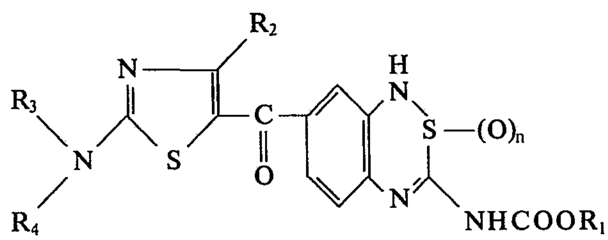

(VII)

dans laquelle $R_1$, $R_2$, $R_3$ et $R_4$ ont les significations indiquées ci-dessus et n est égal à 0 ou à 1, par un acide, ou bien

d) on cyclise un ester o-aminothioallophanique de formule VIII

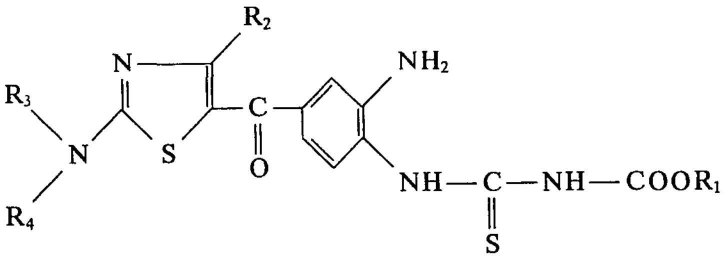

(VIII)

dans laquelle $R_1$, $R_2$, $R_3$ et $R_4$ ont les significations indiquées en référence à la formule I, en présence d'un acide, de préférence un acide minéral, ou bien

e) dans des composés de formule IX

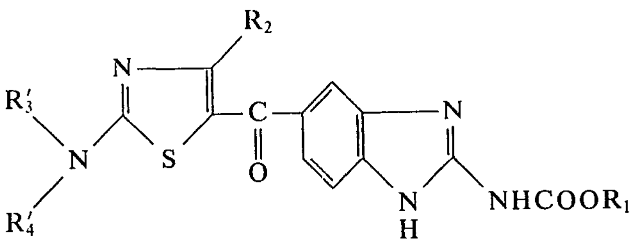

(IX)

dans laquelle $R_1$ et $R_2$ ont les significations indiquées ci-dessus, et l'un des deux substituants $R_3'$ et $R_4'$ représente l'hydrogène ou a la même signification que $R_3$ ou $R_4$, et l'autre représente un groupe protecteur du groupe amino, on élimine ce groupe protecteur, et, si on le désire, on convertit un composé obtenu répondant à la formule I en un autre composé de formule I, et/ou, si on le désire, on convertit un sel obtenu en le composé libre ou en un autre sel, et/ou, si on le désire, on convertit un composé libre obtenu en un sel, et/ou, si on le désire, on sépare un mélange d'isomères obtenu en les isomères individuels.

9. Les composés qu'on peut obtenir par le procédé de la revendication 8.